# EUROPEAN PATENT APPLICATION

(11) **EP 1 118 860 A1**
(43) Date of publication of application: **25.07.2001**
(21) Application number: 00200242.6
(22) Date of filing: 21.01.2000
(51) Int. Cl.: G01N 33/53, C12Q 1/37, A61K 39/00

(54) **Methods for selecting and producing T cell peptide epitopes and vaccines incorporating said selected epitopes**

(71) Applicant: Rijksuniversiteit te Leiden, 2312 AV Leiden (NL); SEED CAPITAL INVESTMENTS( SCI) B.V., 3527 GA Utrecht (NL)
(72) Inventor: Ossendorp, Ferry, 1182 CT Amstelveen (NL); Offringa, Rienk, 2312 SJ Leiden (NL); Melief, Cornelis Johannes Maria, 2012 KC Haarlem (NL); Kessler, Johan Herman, 1091 SC Amsterdam (NL)
(74) Representative: Ottevangers, Sietse Ulbe

(57) **Abstract**

We systematically investigated proteasome-mediated generation of fourteen different well-defined CTL epitopes. Synthetic peptides (26 residues) containing known CTL-epitopes flanked by their natural amino acids have been used as substrates for the 20S proteasome in vitro. After several time intervals, peptide digests were analyzed by electrospray mass spectrometry to determine the major fragments produced by the proteasome. In 12 out of 14 peptide digests, the correct C-terminal residue of the CTL-epitope was generated by proteasomal cleavage. The N-terminal residue of the epitope was generally not exactly defined by the proteasome. In most cases, fragments with the correct C-terminal residue were elongated several amino acids at the N-terminus. For two CTL-epitopes we found that their longer precursor peptides, as generated by the proteasome, correlated with efficient TAP translocation. For one CTL-epitope we found that a natural mutation directly flanking the C-terminal residue of the CTL-epitope precursor disrupted the specific C-terminal cleavage site and resulted in a non-functional cleavage product. This study indicates that proper CTL-epitope generation requires correct C-terminal cleavage by the proteasome, and allows N-terminal elongation of CTL-epitope precursor peptides.

### Annex to the application documents - subsquently filed sequences listing

SEQUENCE LISTING

<110> Rijksuniversiteit te Leiden

<120> Methods for selecting and producing T cell peptide epitopes and vaccines
incorporating said selected epitopes

<130> P52254EP00

<140> 00200242.6
<141> 2000-01-21

<160> 202

<170> PatentIn Ver. 2.1

<210> 1
<211> 26
<212> PRT
<213> Artificial Sequence

<220>
<223> Description of Artificial Sequence: MCF env-p15E

<220>
<221> PEPTIDE
<222> (1)..(26)

<400> 1
Phe Glu Ser Gln Gln Gly Trp Phe Glu Gly Leu Phe Asn Lys Ser Pro
1               5                   10                  15

Trp Phe Thr Thr Leu Ile Ser Thr Ile Met
            20                  25

<210> 2
<211> 26
<212> PRT
<213> Artificial Sequence

<220>
<223> Description of Artificial Sequence: HPV E7

<220>
<221> PEPTIDE
<222> (1)..(26)

<400> 2
Asp Gly Pro Ala Gly Gln Ala Glu Pro Asp Arg Ala His Tyr Asn Ile
1               5                   10                  15

Val Thr Phe Cys Cys Lys Cys Asp Ser Thr
            20                  25

<210> 3
<211> 26
<212> PRT
<213> Artificial Sequence

<220>
<223> Description of Artificial Sequence: E. coli P9

<220>
<221> PEPTIDE
<222> (1)..(26)

<400> 3
Arg Glu Ala Arg Val Ser Ser Ser Pro Val Asn Ser Leu Arg Asn Val
  1               5                  10                  15

Val Ala Ile Ala Ala Gly Ile Val Val Ser
            20                  25

<210> 4
<211> 26
<212> PRT
<213> Artificial Sequence

<220>
<223> Description of Artificial Sequence: Sendai NP

<220>
<221> PEPTIDE
<222> (1)..(26)

<400> 4
Ile Leu Lys Asp Pro Val His Gly Glu Phe Ala Pro Gly Asn Tyr Pro
  1               5                  10                  15

Ala Leu Trp Ser Tyr Ala Met Gly Val Ala
            20                  25

<210> 5
<211> 26
<212> PRT
<213> Artificial Sequence

<220>
<223> Description of Artificial Sequence: MuLV (Moloney)

<220>
<221> PEPTIDE
<222> (1)..(26)

<400> 5
Thr Gly Arg Ala Tyr Trp Lys Pro Ser Ser Ser Trp Asp Phe Ile Thr
1               5                   10                  15

Val Asn Asn Asn Leu Thr Ser Asp Gln Ala
            20                  25

<210> 6
<211> 26
<212> PRT
<213> Artificial Sequence

<220>
<223> Description of Artificial Sequence: Measles NP

<220>
<221> PEPTIDE
<222> (1)..(26)

<400> 6
Lys Phe Gly Ile Glu Thr Met Tyr Pro Ala Leu Gly Leu His Glu Phe
1               5                   10                  15

Ala Gly Glu Leu Ser Thr Leu Glu Ser Leu
            20                  25

<210> 7
<211> 26
<212> PRT
<213> Artificial Sequence

<220>
<223> Description of Artificial Sequence: Measles HA

<220>
<221> PEPTIDE
<222> (1)..(26)

<400> 7
Ser Trp Val Thr Leu Ser Thr Asp Asp Pro Val Ile Asp Arg Leu Tyr

1          5               10               15

Leu Ser Ser His Arg Gly Val Ile Ala Asp
          20               25

<210> 8
<211> 26
<212> PRT
<213> Artificial Sequence

<220>
<223> Description of Artificial Sequence: P53

<220>
<221> PEPTIDE
<222> (1)..(26)

<400> 8
Ala Gly Ser Arg Val Arg Ala Met Ala Ile Tyr Lys Lys Ser Gln His
1          5               10               15

Met Thr Glu Val Val Arg Arg Cys Pro His
          20               25

<210> 9
<211> 26
<212> PRT
<213> Artificial Sequence

<220>
<223> Description of Artificial Sequence: Rotavirus VP-6

<220>
<221> PEPTIDE
<222> (1)..(26)

<400> 9
Arg Gln Glu Tyr Ala Ile Pro Val Gly Pro Val Phe Pro Pro Gly Met
1          5               10               15

Asn Trp Thr Glu Leu Ile Thr Asn Tyr Ser
          20               25

<210> 10
<211> 26
<212> PRT

<213> Artificial Sequence

<220>
<223> Description of Artificial Sequence: P53

<220>
<221> PEPTIDE
<222> (1)..(26)

<400> 10
Tyr Glu Pro Pro Glu Ala Gly Ser Glu Tyr Thr Thr Ile His Tyr Lys
1               5                   10                  15

Tyr Met Cys Asn Ser Ser Cys Met Gly Gly
            20                  25

<210> 11
<211> 26
<212> PRT
<213> Artificial Sequence

<220>
<223> Description of Artificial Sequence: Influenza NP

<220>
<221> PEPTIDE
<222> (1)..(26)

<400> 11
Gly Glu Asn Gly Arg Lys Thr Arg Ser Ala Tyr Glu Arg Met Cys Asn
1               5                   10                  15

Ile Leu Lys Gly Lys Phe Gln Thr Ala Ala
            20                  25

<210> 12
<211> 26
<212> PRT
<213> Artificial Sequence

<220>
<223> Description of Artificial Sequence: Tum, P198

<220>
<221> PEPTIDE
<222> (1)..(26)

<400> 12

Gly Arg Leu Ala His Glu Val Glu Trp Lys Tyr Gln Ala Val Thr Thr
1               5                   10                  15

Thr Leu Glu Glu Lys Arg Lys Glu Lys Ala
            20                  25

<210> 13
<211> 26
<212> PRT
<213> Artificial Sequence

<220>
<223> Description of Artificial Sequence: SV40 T

<220>
<221> PEPTIDE
<222> (1)..(26)

<400> 13
Gly Glu Ser Arg Asp Leu Pro Ser Gly Gln Gly Ile Asn Asn Leu Asp
1               5                   10                  15

Asn Leu Arg Asp Tyr Leu Asp Gly Ser Val
            20                  25

<210> 14
<211> 26
<212> PRT
<213> Artificial Sequence

<220>
<223> Description of Artificial Sequence: Cyclin D1

<220>
<221> PEPTIDE
<222> (1)..(26)

<400> 14
Thr Ile Arg Arg Ala Tyr Pro Asp Thr Asn Leu Leu Asn Asp Arg Val
1               5                   10                  15

Leu Arg Ala Met Leu Lys Thr Glu Glu Thr
            20                  25

<210> 15
<211> 18

<212> PRT
<213> Sendai

<220>
<221> PEPTIDE
<222> (1)..(18)

<400> 15
Ile Leu Lys Asp Pro Val His Gly Glu Phe Ala Pro Gly Asn Tyr Pro
1               5                   10                  15

Ala Leu

<210> 16
<211> 11
<212> PRT
<213> Sendai

<220>
<221> PEPTIDE
<222> (1)..(11)

<400> 16
Gly Glu Phe Ala Pro Gly Asn Tyr Pro Ala Leu
1               5                   10

<210> 17
<211> 7
<212> PRT
<213> Sendai

<220>
<221> PEPTIDE
<222> (1)..(7)

<400> 17
Pro Gly Asn Tyr Pro Ala Leu
1               5

<210> 18
<211> 14
<212> PRT
<213> Sendai

<220>

<221> PEPTIDE
<222> (1)..(14)

<400> 18
Pro Val His Gly Glu Phe Ala Pro Gly Asn Tyr Pro Ala Leu
1               5                   10

<210> 19
<211> 8
<212> PRT
<213> Sendai

<220>
<221> PEPTIDE
<222> (1)..(8)

<400> 19
Ala Pro Gly Asn Tyr Pro Ala Leu
1               5

<210> 20
<211> 10
<212> PRT
<213> Sendai

<220>
<221> PEPTIDE
<222> (1)..(10)

<400> 20
Glu Phe Ala Pro Gly Asn Tyr Pro Ala Leu
1               5                   10

<210> 21
<211> 9
<212> PRT
<213> Sendai

<220>
<221> PEPTIDE
<222> (1)..(9)

<400> 21
Phe Ala Pro Gly Asn Tyr Pro Ala Leu
1               5

<210> 22
<211> 11
<212> PRT
<213> Sendai

<220>
<221> PEPTIDE
<222> (1)..(11)

<400> 22
Lys Asp Pro Val His Gly Glu Phe Ala Pro Gly
1               5                   10

<210> 23
<211> 13
<212> PRT
<213> Sendai

<220>
<221> PEPTIDE
<222> (1)..(13)

<400> 23
Ile Leu Lys Asp Pro Val His Gly Glu Phe Ala Pro Gly
1               5                   10

<210> 24
<211> 15
<212> PRT
<213> MCF

<220>
<221> PEPTIDE
<222> (1)..(15)

<400> 24
Phe Glu Ser Gln Gln Gly Trp Phe Glu Gly Leu Phe Asn Lys Ser
1               5                   10                  15

<210> 25
<211> 6
<212> PRT
<213> MCF

<220>
<221> PEPTIDE
<222> (1)..(6)

<400> 25
Leu Ile Ser Thr Ile Met
1               5

<210> 26
<211> 6
<212> PRT
<213> MCF

<220>
<221> PEPTIDE
<222> (1)..(6)

<400> 26
Pro Trp Phe Thr Thr Leu
1               5

<210> 27
<211> 7
<212> PRT
<213> MCF

<220>
<221> PEPTIDE
<222> (1)..(7)

<400> 27
Pro Trp Phe Thr Thr Leu Ile
1               5

<210> 28
<211> 10
<212> PRT
<213> MCF

<220>
<221> PEPTIDE
<222> (1)..(10)

<400> 28
Pro Trp Phe Thr Thr Leu Ile Ser Thr Ile
1               5                   10

<210> 29
<211> 14
<212> PRT
<213> MCF

<220>
<221> PEPTIDE
<222> (1)..(14)

<400> 29
Phe Asn Lys Ser Pro Trp Phe Thr Thr Leu Ile Ser Thr Ile
1               5                   10

<210> 30
<211> 11
<212> PRT
<213> MCF

<220>
<221> PEPTIDE
<222> (1)..(11)

<400> 30
Phe Glu Ser Gln Gln Gly Trp Phe Glu Gly Leu
1               5                   10

<210> 31
<211> 21
<212> PRT
<213> MCF

<220>
<221> PEPTIDE
<222> (1)..(21)

<400> 31
Phe Glu Ser Gln Gln Gly Trp Phe Glu Gly Leu Phe Asn Lys Ser Pro
1               5                   10                  15

Trp Phe Thr Thr Leu
            20

<210> 32

<211> 15
<212> PRT
<213> MCF

<220>
<221> PEPTIDE
<222> (1)..(15)

<400> 32
Phe Asn Lys Ser Pro Trp Phe Thr Thr Leu Ile Ser Thr Ile Met
1               5                   10                  15

<210> 33
<211> 11
<212> PRT
<213> MCF

<220>
<221> PEPTIDE
<222> (1)..(11)

<400> 33
Phe Asn Lys Ser Pro Trp Phe Thr Thr Leu Ile
1               5                   10

<210> 34
<211> 20
<212> PRT
<213> MCF

<220>
<221> PEPTIDE
<222> (1)..(20)

<400> 34
Phe Glu Ser Gln Gln Gly Trp Phe Glu Gly Leu Phe Asn Lys Ser Pro
1               5                   10                  15

Trp Phe Thr Thr
            20

<210> 35
<211> 10
<212> PRT
<213> MCF

<220>
<221> PEPTIDE
<222> (1)..(10)

<400> 35
Phe Asn Lys Ser Pro Trp Phe Thr Thr Leu
1               5                   10

<210> 36
<211> 15
<212> PRT
<213> MCF

<220>
<221> PEPTIDE
<222> (1)..(15)

<400> 36
Phe Glu Ser Gln Gln Gly Trp Phe Glu Gly Leu Phe Asn Lys Ser
1               5                   10                  15

<210> 37
<211> 7
<212> PRT
<213> Moloney

<220>
<221> PEPTIDE
<222> (1)..(7)

<400> 37
Ser Trp Asp Phe Ile Thr Val
1               5

<210> 38
<211> 10
<212> PRT
<213> Moloney

<220>
<221> PEPTIDE
<222> (1)..(10)

<400> 38
Pro Ser Ser Ser Trp Asp Phe Ile Thr Val
1               5                   10

<210> 39
<211> 17
<212> PRT
<213> Moloney

<220>
<221> PEPTIDE
<222> (1)..(17)

<400> 39
Thr Gly Arg Ala Tyr Trp Lys Pro Ser Ser Ser Asp Trp Phe Ile Thr
1               5                   10                  15

Val

<210> 40
<211> 14
<212> PRT
<213> Moloney

<220>
<221> PEPTIDE
<222> (1)..(14)

<400> 40
Ala Tyr Trp Lys Pro Ser Ser Ser Trp Asp Phe Ile Thr Val
1               5                   10

<210> 41
<211> 26
<212> PRT
<213> AKV

<220>
<221> PEPTIDE
<222> (1)..(26)

<400> 41
Thr Gly Arg Ala Ser Trp Lys Pro Ser Ser Ser Trp Asp Tyr Ile Thr
1               5                   10                  15

Val Ser Asn Asn Leu Thr Ser Asp Gln Ala
            20                  25

<210> 42
<211> 23
<212> PRT
<213> AKV

<220>
<221> PEPTIDE
<222> (1)..(23)

<400> 42
Ala Ser Trp Lys Pro Ser Ser Ser Trp Asp Tyr Ile Thr Val Ser Asn
1               5                   10                  15

Asn Leu Thr Ser Asp Gln Ala
            20

<210> 43
<211> 17
<212> PRT
<213> AKV

<220>
<221> PEPTIDE
<222> (1)..(17)

<400> 43
Thr Gly Arg Ala Ser Trp Lys Pro Ser Ser Ser Trp Asp Tyr Ile Thr
1               5                   10                  15

Val

<210> 44
<211> 7
<212> PRT
<213> AKV

<220>
<221> PEPTIDE
<222> (1)..(7)

<400> 44
Ser Trp Asp Tyr Ile Thr Val
1               5

<210> 45
<211> 14
<212> PRT
<213> AKV

<220>
<221> PEPTIDE
<222> (1)..(14)

<400> 45
Ala Ser Trp Lys Pro Ser Ser Ser Trp Asp Tyr Ile Thr Val
1               5                   10

<210> 46
<211> 10
<212> PRT
<213> AKV

<220>
<221> PEPTIDE
<222> (1)..(10)

<400> 46
Pro Ser Ser Ser Trp Asp Tyr Ile Thr Val
1               5                   10

<210> 47
<211> 26
<212> PRT
<213> Friend MuLV

<220>
<221> PEPTIDE
<222> (1)..(26)

<400> 47
Thr Gly Arg Ala Tyr Trp Lys Pro Ser Ser Ser Trp Asp Tyr Ile Thr
1               5                   10                  15

Val Asp Asn Asn Leu Thr Thr Asn Gln Ala
            20                  25

<210> 48
<211> 18
<212> PRT

<213> Friend MuLV

<220>
<221> PEPTIDE
<222> (1)..(18)

<400> 48
Thr Gly Arg Ala Tyr Trp Lys Pro Ser Ser Ser Trp Asp Tyr Ile Thr
1               5                   10                  15

Val Asp

<210> 49
<211> 15
<212> PRT
<213> Friend MuLV

<220>
<221> PEPTIDE
<222> (1)..(15)

<400> 49
Ala Tyr Trp Lys Pro Ser Ser Ser Trp Asp Tyr Ile Thr Val Asp
1               5                   10                  15

<210> 50
<211> 23
<212> PRT
<213> Friend MuLV

<220>
<221> PEPTIDE
<222> (1)..(23)

<400> 50
Ala Tyr Trp Lys Pro Ser Ser Ser Trp Asp Tyr Ile Thr Val Asp Asn
1               5                   10                  15

Asn Leu Thr Thr Asn Gln Ala
            20

<210> 51
<211> 19
<212> PRT
<213> Friend MuLV

<220>
<221> PEPTIDE
<222> (1)..(19)

<400> 51
Pro Ser Ser Ser Trp Asp Tyr Ile Thr Val Asp Asn Asn Leu Thr Thr
1               5                   10                  15

Asn Gln Ala

<210> 52
<211> 8
<212> PRT
<213> Friend MuLV

<220>
<221> PEPTIDE
<222> (1)..(8)

<400> 52
Ser Trp Asp Tyr Ile Thr Val Asp
1               5

<210> 53
<211> 11
<212> PRT
<213> Friend MuLV

<220>
<221> PEPTIDE
<222> (1)..(11)

<400> 53
Pro Ser Ser Ser Trp Asp Tyr Ile Thr Val Asp
1               5                   10

<210> 54
<211> 10
<212> PRT
<213> Artificial Sequence

<220>
<223> Description of Artificial Sequence: peptide

<220>
<221> PEPTIDE
<222> (1)..(10)
<223> /note="Start 300"

<400> 54
Ala Leu Tyr Val Asp Ser Leu Phe Phe Leu
1               5                   10

<210> 55
<211> 10
<212> PRT
<213> Artificial Sequence

<220>
<223> Description of Artificial Sequence: peptide

<220>
<221> PEPTIDE
<222> (1)..(10)
<223> /note="Start 142"

<400> 55
Ser Leu Tyr Ser Phe Pro Glu Pro Glu Ala
1               5                   10

<210> 56
<211> 10
<212> PRT
<213> Artificial Sequence

<220>
<223> Description of Artificial Sequence: peptide

<220>
<221> PEPTIDE
<222> (1)..(10)
<223> /note="Start 47"

<400> 56
Leu Leu Pro Arg Glu Leu Phe Pro Pro Leu
1               5                   10

<210> 57
<211> 9
<212> PRT

<213> Artificial Sequence

<220>
<223> Description of Artificial Sequence: peptide

<220>
<221> PEPTIDE
<222> (1)..(9)
<223> /note="Start 435"

<400> 57
Asn Leu Thr His Val Leu Tyr Pro Val
1               5

<210> 58
<211> 10
<212> PRT
<213> Artificial Sequence

<220>
<223> Description of Artificial Sequence: peptide

<220>
<221> PEPTIDE
<222> (1)..(10)
<223> /note="Start 292"

<400> 58
Phe Leu Ser Leu Gln Cys Leu Gln Ala Leu
1               5                   10

<210> 59
<211> 9
<212> PRT
<213> Artificial Sequence

<220>
<223> Description of Artificial Sequence: peptide

<220>
<221> PEPTIDE
<222> (1)..(9)
<223> /note="Start 394"

<400> 59
Gln Leu Leu Ala Leu Leu Pro Ser Leu
1               5

<210> 60
<211> 10
<212> PRT
<213> Artificial Sequence

<220>
<223> Description of Artificial Sequence: peptide

<220>
<221> PEPTIDE
<222> (1)..(10)
<223> /note="Start 182"

<400> 60
Phe Leu Lys Glu Gly Ala Cys Asp Glu Leu
1               5                   10

<210> 61
<211> 10
<212> PRT
<213> Artificial Sequence

<220>
<223> Description of Artificial Sequence: peptide

<220>
<221> PEPTIDE
<222> (1)..(10)
<223> /note="Start 294"

<400> 61
Ser Leu Gln Cys Leu Gln Ala Leu Tyr Val
1               5                   10

<210> 62
<211> 10
<212> PRT
<213> Artificial Sequence

<220>
<223> Description of Artificial Sequence: peptide

<220>
<221> PEPTIDE
<222> (1)..(10)
<223> /note="Start 422"

<400> 62
Ala Leu Gln Ser Leu Leu Gln His Leu Ile
1               5                   10

<210> 63
<211> 9
<212> PRT
<213> Artificial Sequence

<220>
<223> Description of Artificial Sequence: peptide

<220>
<221> PEPTIDE
<222> (1)..(9)
<223> /note="Start 425"

<400> 63
Ser Leu Leu Gln His Leu Ile Gly Leu
1               5

<210> 64
<211> 10
<212> PRT
<213> Artificial Sequence

<220>
<223> Description of Artificial Sequence: peptide

<220>
<221> PEPTIDE
<222> (1)..(10)
<223> /note="Start 258"

<400> 64
Gln Met Ile Asn Leu Arg Arg Leu Leu Leu
1               5                   10

<210> 65
<211> 10
<212> PRT
<213> Artificial Sequence

<220>
<223> Description of Artificial Sequence: peptide

<220>
<221> PEPTIDE
<222> (1)..(10)
<223> /note="Start 190"

<400> 65
Glu Leu Phe Ser Tyr Leu Ile Glu Lys Val
1               5                   10

<210> 66
<211> 9
<212> PRT
<213> Artificial Sequence

<220>
<223> Description of Artificial Sequence: peptide

<220>
<221> PEPTIDE
<222> (1)..(9)
<223> /note="Start 248"

<400> 66
Thr Leu Ala Lys Phe Ser Pro Tyr Leu
1               5

<210> 67
<211> 9
<212> PRT
<213> Artificial Sequence

<220>
<223> Description of Artificial Sequence: peptide

<220>
<221> PEPTIDE
<222> (1)..(9)
<223> /note="Start 39"

<400> 67
Ala Leu Ala Ile Ala Ala Leu Glu Leu
1               5

<210> 68
<211> 9

<212> PRT
<213> Artificial Sequence

<220>
<223> Description of Artificial Sequence: peptide

<220>
<221> PEPTIDE
<222> (1)..(9)
<223> /note="Start 100"

<400> 68
Val Leu Asp Gly Leu Asp Val Leu Leu
1               5

<210> 69
<211> 10
<212> PRT
<213> Artificial Sequence

<220>
<223> Description of Artificial Sequence: peptide

<220>
<221> PEPTIDE
<222> (1)..(10)
<223> /note="Start 333"

<400> 69
Arg Leu Ser Glu Gly Asp Val Met His Leu
1               5                   10

<210> 70
<211> 9
<212> PRT
<213> Artificial Sequence

<220>
<223> Description of Artificial Sequence: peptide

<220>
<221> PEPTIDE
<222> (1)..(9)
<223> /note="Start 462"

<400> 70
Tyr Leu His Ala Arg Leu Arg Glu Leu
1               5

<210> 71
<211> 10
<212> PRT
<213> Artificial Sequence

<220>
<223> Description of Artificial Sequence: peptide

<220>
<221> PEPTIDE
<222> (1)..(10)
<223> /note="Start 360"

<400> 71
Met Leu Thr Asp Val Ser Pro Glu Pro Leu
1               5                   10

<210> 72
<211> 9
<212> PRT
<213> Artificial Sequence

<220>
<223> Description of Artificial Sequence: peptide

<220>
<221> PEPTIDE
<222> (1)..(9)
<223> /note="Start 419"

<400> 72
Ser Ile Ser Ala Leu Gln Ser Leu Leu
1               5

<210> 73
<211> 9
<212> PRT
<213> Artificial Sequence

<220>
<223> Description of Artificial Sequence: peptide

<220>
<221> PEPTIDE
<222> (1)..(9)

<223> /note="Start 432"

<400> 73
Gly Leu Ser Asn Leu Thr His Val Leu
1               5

<210> 74
<211> 10
<212> PRT
<213> Artificial Sequence

<220>
<223> Description of Artificial Sequence: peptide

<220>
<221> PEPTIDE
<222> (1)..(10)
<223> /note="Start 214"

<400> 74
Lys Ile Phe Ala Met Pro Met Gln Asp Ile
1               5                   10

<210> 75
<211> 10
<212> PRT
<213> Artificial Sequence

<220>
<223> Description of Artificial Sequence: peptide

<220>
<221> PEPTIDE
<222> (1)..(10)
<223> /note="Start 320"

<400> 75
Val Met Asn Pro Leu Glu Thr Leu Ser Ile
1               5                   10

<210> 76
<211> 10
<212> PRT
<213> Artificial Sequence

<220>

<223> Description of Artificial Sequence: peptide

<220>
<221> PEPTIDE
<222> (1)..(10)
<223> /note="Start 39"

<400> 76
Ala Leu Ala Ile Ala Ala Leu Glu Leu Leu
1               5                   10

<210> 77
<211> 9
<212> PRT
<213> Artificial Sequence

<220>
<223> Description of Artificial Sequence: peptide

<220>
<221> PEPTIDE
<222> (1)..(9)
<223> /note="Start 390"

<400> 77
Ile Thr Asp Asp Gln Leu Leu Ala Leu
1               5

<210> 78
<211> 9
<212> PRT
<213> Artificial Sequence

<220>
<223> Description of Artificial Sequence: peptide

<220>
<221> PEPTIDE
<222> (1)..(9)
<223> /note="Start 242"

<400> 78
Cys Thr Trp Lys Leu Pro Thr Leu Ala
1               5

<210> 79

<211> 10
<212> PRT
<213> Artificial Sequence

<220>
<223> Description of Artificial Sequence: peptide

<220>
<221> PEPTIDE
<222> (1)..(10)
<223> /note="Start 99"

<400> 79
Ala Val Leu Asp Gly Leu Asp Val Leu Leu
1               5                   10

<210> 80
<211> 10
<212> PRT
<213> Artificial Sequence

<220>
<223> Description of Artificial Sequence: peptide

<220>
<221> PEPTIDE
<222> (1)..(10)
<223> note="Start 308"

<400> 80
Phe Leu Arg Gly Arg Leu Asp Gln Leu Leu
1               5                   10

<210> 81
<211> 10
<212> PRT
<213> Artificial Sequence

<220>
<223> Description of Artificial Sequence: peptide

<220>
<221> PEPTIDE
<222> (1)..(10)
<223> /note="Start 355"

<400> 81
Ser Leu Ser Gly Val Met Leu Thr Asp Val

1          5               10

<210> 82
<211> 9
<212> PRT
<213> Artificial Sequence

<220>
<223> Description of Artificial Sequence: peptide

<220>
<221> PEPTIDE
<222> (1)..(9)
<223> /note="Start 34"

<400> 82
Leu Leu Lys Asp Glu Ala Leu Ala Ile
 1          5

<210> 83
<211> 10
<212> PRT
<213> Artificial Sequence

<220>
<223> Description of Artificial Sequence: peptide

<220>
<221> PEPTIDE
<222> (1)..(10)
<223> /note="Start 284"

<400> 83
Tyr Ile Ala Gln Phe Thr Ser Gln Phe Leu
 1          5               10

<210> 84
<211> 10
<212> PRT
<213> Artificial Sequence

<220>
<223> Description of Artificial Sequence: peptide

<220>
<221> PEPTIDE

<222> (1)..(10)
<223> /note="Start 71"

<400> 84
Ala Met Val Gln Ala Trp Pro Phe Thr Cys
1               5                   10

<210> 85
<211> 10
<212> PRT
<213> Artificial Sequence

<220>
<223> Description of Artificial Sequence: peptide

<220>
<221> PEPTIDE
<222> (1)..(10)
<223> /note="Start 470"

<400> 85
Leu Leu Cys Glu Leu Gly Arg Pro Ser Met
1               5                   10

<210> 86
<211> 10
<212> PRT
<213> Artificial Sequence

<220>
<223> Description of Artificial Sequence: peptide

<220>
<221> PEPTIDE
<222> (1)..(10)
<223> /note="Start 186"

<400> 86
Gly Ala Cys Asp Glu Leu Phe Ser Tyr Leu
1               5                   10

<210> 87
<211> 9
<212> PRT
<213> Artificial Sequence

<220>
<223> Description of Artificial Sequence: peptide

<220>
<221> PEPTIDE
<222> (1)..(9)
<223> /note="Start 410"

<400> 87
Thr Leu Ser Phe Tyr Gly Asn Ser Ile
1               5

<210> 88
<211> 10
<212> PRT
<213> Artificial Sequence

<220>
<223> Description of Artificial Sequence: peptide

<220>
<221> PEPTIDE
<222> (1)..(10)
<223> /note="Start 25"

<400> 88
Arg Leu Val Glu Leu Ala Gly Gln Ser Leu
1               5                   10

<210> 89
<211> 9
<212> PRT
<213> Artificial Sequence

<220>
<223> Description of Artificial Sequence: peptide

<220>
<221> PEPTIDE
<222> (1)..(9)
<223> /note="Start 91"

<400> 89
His Leu His Leu Glu Thr Phe Lys Ala
1               5

<210> 90
<211> 10
<212> PRT
<213> Artificial Sequence

<220>
<223> Description of Artificial Sequence: peptide

<220>
<221> PEPTIDE
<222> (1)..(10)
<223> /note="Start 100"

<400> 90
Val Leu Asp Gly Leu Asp Val Leu Leu Ala
1               5                   10

<210> 91
<211> 10
<212> PRT
<213> Artificial Sequence

<220>
<223> Description of Artificial Sequence: peptide

<220>
<221> PEPTIDE
<222> (1)..(10)
<223> /note="Start 454"

<400> 91
Thr Leu His Leu Glu Arg Leu Ala Tyr Leu
1               5                   10

<210> 92
<211> 10
<212> PRT
<213> Artificial Sequence

<220>
<223> Description of Artificial Sequence: peptide

<220>
<221> PEPTIDE
<222> (1)..(10)
<223> /note="Start 371"

<400> 92

Ala Leu Leu Glu Arg Ala Ser Ala Thr Leu
1               5                    10

<210> 93
<211> 9
<212> PRT
<213> Artificial Sequence

<220>
<223> Description of Artificial Sequence: peptide

<220>
<221> PEPTIDE
<222> (1)..(9)
<223> /note="Start 326"

<400> 93
Thr Leu Ser Ile Thr Asn Cys Arg Leu
1               5

<210> 94
<211> 10
<212> PRT
<213> Artificial Sequence

<220>
<223> Description of Artificial Sequence: peptide

<220>
<221> PEPTIDE
<222> (1)..(10)
<223> /note="Start 462"

<400> 94
Tyr Leu His Ala Arg Leu Arg Glu Leu Leu
1               5                    10

<210> 95
<211> 10
<212> PRT
<213> Artificial Sequence

<220>
<223> Description of Artificial Sequence: peptide

<220>

<221> PEPTIDE
<222> (1)..(10)
<223> /note="Start 350"

<400> 95
Gln Leu Ser Val Leu Ser Leu Ser Gly Val
1               5                   10

<210> 96
<211> 9
<212> PRT
<213> Artificial Sequence

<220>
<223> Description of Artificial Sequence: peptide

<220>
<221> PEPTIDE
<222> (1)..(9)
<223> /note="Start 99"

<400> 96
Ala Val Leu Asp Gly Leu Asp Val Leu
1               5

<210> 97
<211> 9
<212> PRT
<213> Artificial Sequence

<220>
<223> Description of Artificial Sequence: peptide

<220>
<221> PEPTIDE
<222> (1)..(9)
<223> /note="Start 466"

<400> 97
Arg Leu Arg Glu Leu Leu Cys Glu Leu
1               5

<210> 98
<211> 10
<212> PRT
<213> Artificial Sequence

<220>
<223> Description of Artificial Sequence: peptide

<220>
<221> PEPTIDE
<222> (1)..(10)
<223> /note="Start 33"

<400> 98
Ser Leu Leu Lys Asp Glu Ala Leu Ala Ile
1               5                   10

<210> 99
<211> 9
<212> PRT
<213> Artificial Sequence

<220>
<223> Description of Artificial Sequence: peptide

<220>
<221> PEPTIDE
<222> (1)..(9)
<223> /note="Start 422"

<400> 99
Ala Leu Gln Ser Leu Leu Gln His Leu
1               5

<210> 100
<211> 10
<212> PRT
<213> Artificial Sequence

<220>
<223> Description of Artificial Sequence: peptide

<220>
<221> PEPTIDE
<222> (1)..(10)
<223> /note="Start 103"

<400> 100
Gly Leu Asp Val Leu Leu Ala Gln Glu Val
1               5                   10

<210> 101
<211> 10
<212> PRT
<213> Artificial Sequence

<220>
<223> Description of Artificial Sequence: peptide

<220>
<221> PEPTIDE
<222> (1)..(10)
<223> note="Start 231"

<400> 101
Gln Leu Asp Ser Ile Glu Asp Leu Glu Val
1               5                   10

<210> 102
<211> 9
<212> PRT
<213> Artificial Sequence

<220>
<223> Description of Artificial Sequence: peptide

<220>
<221> PEPTIDE
<222> (1)..(9)
<223> /note="Start 312"

<400> 102
Arg Leu Asp Gln Leu Leu Arg His Val
1               5

<210> 103
<211> 9
<212> PRT
<213> Artificial Sequence

<220>
<223> Description of Artificial Sequence: peptide

<220>
<221> PEPTIDE
<222> (1)..(9)
<223> /note="Start 493"

<400> 103
Arg Thr Phe Tyr Asp Pro Glu Pro Ile
1               5

<210> 104
<211> 9
<212> PRT
<213> Artificial Sequence

<220>
<223> Description of Artificial Sequence: peptide

<220>
<221> PEPTIDE
<222> (1)..(9)
<223> /note="Start 308"

<400> 104
Phe Leu Arg Gly Arg Leu Asp Gln Leu
1               5

<210> 105
<211> 9
<212> PRT
<213> Artificial Sequence

<220>
<223> Description of Artificial Sequence: peptide

<220>
<221> PEPTIDE
<222> (1)..(9)
<223> /note="Start 429"

<400> 105
His Leu Ile Gly Leu Ser Asn Leu Thr
1               5

<210> 106
<211> 10
<212> PRT
<213> Artificial Sequence

<220>
<223> Description of Artificial Sequence: peptide

<220>
<221> PEPTIDE
<222> (1)..(10)
<223> /note="Start 85"

<400> 106
Val Leu Met Lys Gly Gln His Leu His Leu
1               5                   10

<210> 107
<211> 9
<212> PRT
<213> Artificial Sequence

<220>
<223> Description of Artificial Sequence: peptide

<220>
<221> PEPTIDE
<222> (1)..(9)
<223> note="Start 316"

<400> 107
Leu Leu Arg His Val Met Asn Pro Leu
1               5

<210> 108
<211> 9
<212> PRT
<213> Artificial Sequence

<220>
<223> Description of Artificial Sequence: peptide

<220>
<221> PEPTIDE
<222> (1)..(9)
<223> note="Start 353"

<400> 108
Val Leu Ser Leu Ser Gly Val Met Leu
1               5

<210> 109
<211> 10
<212> PRT

<213> Artificial Sequence

<220>
<223> Description of Artificial Sequence: peptide

<220>
<221> PEPTIDE
<222> (1)..(10)
<223> /note="Start 172"

<400> 109
Phe Ile Pro Val Glu Val Leu Val Asp Leu
1               5                   10

<210> 110
<211> 10
<212> PRT
<213> Artificial Sequence

<220>
<223> Description of Artificial Sequence: peptide

<220>
<221> PEPTIDE
<222> (1)..(10)
<223> /note="Start 134"

<400> 110
Thr Val Trp Ser Gly Asn Arg Ala Ser Leu
1               5                   10

<210> 111
<211> 10
<212> PRT
<213> Artificial Sequence

<220>
<223> Description of Artificial Sequence: peptide

<220>
<221> PEPTIDE
<222> (1)..(10)
<223> /note="Start 339"

<400> 111
Val Met His Leu Ser Gln Ser Pro Ser Val
1               5                   10

<210> 112
<211> 9
<212> PRT
<213> Artificial Sequence

<220>
<223> Description of Artificial Sequence: peptide

<220>
<221> PEPTIDE
<222> (1)..(9)
<223> /note="Start 72"

<400> 112
Met Val Gln Ala Trp Pro Phe Thr Cys
1               5

<210> 113
<211> 10
<212> PRT
<213> Artificial Sequence

<220>
<223> Description of Artificial Sequence: peptide

<220>
<221> PEPTIDE
<222> (1)..(10)
<223> /note="Start 390"

<400> 113
Ile Thr Asp Asp Gln Leu Leu Ala Leu Leu
1               5                   10

<210> 114
<211> 9
<212> PRT
<213> Artificial Sequence

<220>
<223> Description of Artificial Sequence: peptide

<220>
<221> PEPTIDE
<222> (1)..(9)
<223> /note="Start 18"

<400> 114
Ser Val Trp Thr Ser Pro Arg Arg Leu
1               5

<210> 115
<211> 10
<212> PRT
<213> Artificial Sequence

<220>
<223> Description of Artificial Sequence: peptide

<220>
<221> PEPTIDE
<222> (1)..(10)
<223> /note="Start 315"

<400> 115
Gln Leu Leu Arg His Val Met Asn Pro Leu
1               5                   10

<210> 116
<211> 9
<212> PRT
<213> Artificial Sequence

<220>
<223> Description of Artificial Sequence: peptide

<220>
<221> PEPTIDE
<222> (1)..(9)
<223> /note="Start 71"

<400> 116
Ala Met Val Gln Ala Trp Pro Phe Thr
1               5

<210> 117
<211> 9
<212> PRT
<213> Artificial Sequence

<220>
<223> Description of Artificial Sequence: peptide

<220>
<221> PEPTIDE
<222> (1)..(9)
<223> /note="Start 207"

<400> 117
Arg Leu Cys Cys Lys Lys Leu Lys Ile
1               5

<210> 118
<211> 10
<212> PRT
<213> Artificial Sequence

<220>
<223> Description of Artificial Sequence: peptide

<220>
<221> PEPTIDE
<222> (1)..(10)
<223> /note="Start 247"

<400> 118
Pro Thr Leu Ala Lys Phe Ser Pro Tyr Leu
1               5                   10

<210> 119
<211> 9
<212> PRT
<213> Artificial Sequence

<220>
<223> Description of Artificial Sequence: peptide

<220>
<221> PEPTIDE
<222> (1)..(9)
<223> /note="Start 219"

<400> 119
Pro Met Gln Asp Ile Lys Met Ile Leu
1               5

<210> 120
<211> 9

<212> PRT
<213> Artificial Sequence

<220>
<223> Description of Artificial Sequence: peptide

<220>
<221> PEPTIDE
<222> (1)..(9)
<223> /note="Start 459"

<400> 120
Arg Leu Ala Tyr Leu His Ala Arg Leu
 1               5

<210> 121
<211> 9
<212> PRT
<213> Artificial Sequence

<220>
<223> Description of Artificial Sequence: peptide

<220>
<221> PEPTIDE
<222> (1)..(9)
<223> /note="Start 264"

<400> 121
Arg Leu Leu Leu Ser His Ile His Ala
 1               5

<210> 122
<211> 10
<212> PRT
<213> Artificial Sequence

<220>
<223> Description of Artificial Sequence: peptide

<220>
<221> PEPTIDE
<222> (1)..(10)
<223> /note="Start 217"

<400> 122
Ala Met Pro Met Gln Asp Ile Lys Met Ile
 1               5                   10

<210> 123
<211> 9
<212> PRT
<213> Artificial Sequence

<220>
<223> Description of Artificial Sequence: peptide

<220>
<221> PEPTIDE
<222> (1)..(9)
<223> /note="Start 361"

<400> 123
Leu Thr Asp Val Ser Pro Glu Pro Leu
1               5

<210> 124
<211> 10
<212> PRT
<213> Artificial Sequence

<220>
<223> Description of Artificial Sequence: peptide

<220>
<221> PEPTIDE
<222> (1)..(10)
<223> /note="Start 430"

<400> 124
Leu Ile Gly Leu Ser Asn Leu Thr His Val
1               5                   10

<210> 125
<211> 9
<212> PRT
<213> Artificial Sequence

<220>
<223> Description of Artificial Sequence: peptide

<220>
<221> PEPTIDE
<222> (1)..(9)

<223> /note="Start 33"

<400> 125
Ser Leu Leu Lys Asp Glu Ala Leu Ala
1               5

<210> 126
<211> 9
<212> PRT
<213> Artificial Sequence

<220>
<223> Description of Artificial Sequence: peptide

<220>
<221> PEPTIDE
<222> (1)..(9)
<223> /note="Start 258"

<400> 126
Gln Met Ile Asn Leu Arg Arg Leu Leu
1               5

<210> 127
<211> 10
<212> PRT
<213> Artificial Sequence

<220>
<223> Description of Artificial Sequence: peptide

<220>
<221> PEPTIDE
<222> (1)..(10)
<223> /note="Start 91"

<400> 127
His Leu His Leu Glu Thr Phe Lys Ala Val
1               5                   10

<210> 128
<211> 10
<212> PRT
<213> Artificial Sequence

<220>

<223> Description of Artificial Sequence: peptide

<220>
<221> PEPTIDE
<222> (1)..(10)
<223> /note="Start 297"

<400> 128
Cys Leu Gln Ala Leu Tyr Val Asp Ser Leu
1               5                   10

<210> 129
<211> 9
<212> PRT
<213> Artificial Sequence

<220>
<223> Description of Artificial Sequence: peptide

<220>
<221> PEPTIDE
<222> (1)..(9)
<223> /note="Start 372"

<400> 129
Leu Leu Glu Arg Ala Ser Ala Thr Leu
1               5

<210> 130
<211> 9
<212> PRT
<213> Artificial Sequence

<220>
<223> Description of Artificial Sequence: peptide

<220>
<221> PEPTIDE
<222> (1)..(9)
<223> /note="Start 401"

<400> 130
Ser Leu Ser His Cys Ser Gln Leu Thr
1               5

<210> 131

<211> 9
<212> PRT
<213> Artificial Sequence

<220>
<223> Description of Artificial Sequence: peptide

<220>
<221> PEPTIDE
<222> (1)..(9)
<223> /note="Start 397"

<400> 131
Ala Leu Leu Pro Ser Leu Ser His Cys
1               5

<210> 132
<211> 10
<212> PRT
<213> Artificial Sequence

<220>
<223> Description of Artificial Sequence: peptide

<220>
<221> PEPTIDE
<222> (1)..(10)
<223> /note="Start 389"

<400> 132
Gly Ile Thr Asp Asp Gln Leu Leu Ala Leu
1               5                   10

<210> 133
<211> 10
<212> PRT
<213> Artificial Sequence

<220>
<223> Description of Artificial Sequence: peptide

<220>
<221> PEPTIDE
<222> (1)..(10)
<223> /note="Start 417"

<400> 133
Ser Ile Ser Ile Ser Ala Leu Gln Ser Leu

1          5               10

<210> 134
<211> 9
<212> PRT
<213> Artificial Sequence

<220>
<223> Description of Artificial Sequence: peptide

<220>
<221> PEPTIDE
<222> (1)..(9)
<223> /note="Start 259"

<400> 134
Met Ile Asn Leu Arg Arg Leu Leu Leu
 1          5

<210> 135
<211> 9
<212> PRT
<213> Artificial Sequence

<220>
<223> Description of Artificial Sequence: peptide

<220>
<221> PEPTIDE
<222> (1)..(9)
<223> /note="Start 479"

<400> 135
Met Val Trp Leu Ser Ala Asn Pro Cys
 1          5

<210> 136
<211> 9
<212> PRT
<213> Artificial Sequence

<220>
<223> Description of Artificial Sequence: peptide

<220>
<221> PEPTIDE

<222> (1)..(9)
<223> /note="Start 160"

<400> 136
Lys Val Asp Gly Leu Ser Thr Glu Ala
1               5

<210> 137
<211> 10
<212> PRT
<213> Artificial Sequence

<220>
<223> Description of Artificial Sequence: peptide

<220>
<221> PEPTIDE
<222> (1)..(10)
<223> /note="Start 436"

<400> 137
Leu Thr His Val Leu Tyr Pro Val Pro Leu
1               5                   10

<210> 138
<211> 10
<212> PRT
<213> Artificial Sequence

<220>
<223> Description of Artificial Sequence: peptide

<220>
<221> PEPTIDE
<222> (1)..(10)
<223> /note="Start 226"

<400> 138
Ile Leu Lys Met Val Gln Leu Asp Ser Ile
1               5                   10

<210> 139
<211> 9
<212> PRT
<213> Artificial Sequence

<220>
<221> PEPTIDE
<222> (1)..(9)
<223> /note="Start 292"

<400> 139
Phe Leu Ser Leu Gln Cys Leu Gln Ala
1               5

<210> 140
<211> 9
<212> PRT
<213> Artificial Sequence

<220>
<223> Description of Artificial Sequence: peptide

<220>
<221> PEPTIDE
<222> (1)..(9)
<223> /note="Start 86"

<400> 140
Leu Met Lys Gly Gln His Leu His Leu
1               5

<210> 141
<211> 10
<212> PRT
<213> Artificial Sequence

<220>
<223> Description of Artificial Sequence: peptide

<220>
<221> PEPTIDE
<222> (1)..(10)
<223> /note="Start 240"

<400> 141
Val Thr Cys Thr Trp Lys Leu Pro Thr Leu
1               5                   10

<210> 142
<211> 9
<212> PRT

<213> Artificial Sequence

<220>
<223> Description of Artificial Sequence: peptide

<220>
<221> PEPTIDE
<222> (1)..(9)
<223> /note="Start 44"

<400> 142
Ala Leu Glu Leu Leu Pro Arg Glu Leu
1               5

<210> 143
<211> 10
<212> PRT
<213> Artificial Sequence

<220>
<223> Description of Artificial Sequence: peptide

<220>
<221> PEPTIDE
<222> (1)..(10)
<223> /note="Start 379"

<400> 143
Thr Leu Gln Asp Leu Val Phe Asp Glu Cys
1               5                   10

<210> 144
<211> 9
<212> PRT
<213> Artificial Sequence

<220>
<223> Description of Artificial Sequence: peptide

<220>
<221> PEPTIDE
<222> (1)..(9)
<223> /note="Start 371"

<400> 144
Ala Leu Leu Glu Arg Ala Ser Ala Thr
1               5

<210> 145
<211> 10
<212> PRT
<213> Artificial Sequence

<220>
<223> Description of Artificial Sequence: peptide

<220>
<221> PEPTIDE
<222> (1)..(10)
<223> /note="Start 353"

<400> 145
Val Leu Ser Leu Ser Gly Val Met Leu Thr
1               5                   10

<210> 146
<211> 9
<212> PRT
<213> Artificial Sequence

<220>
<223> Description of Artificial Sequence: peptide

<220>
<221> PEPTIDE
<222> (1)..(9)
<223> /note="Start 305"

<400> 146
Ser Leu Phe Phe Leu Arg Gly Arg Leu
1               5

<210> 147
<211> 10
<212> PRT
<213> Artificial Sequence

<220>
<223> Description of Artificial Sequence: peptide

<220>
<221> PEPTIDE
<222> (1)..(10)
<223> /note="Start 409"

<400> 147
Thr Thr Leu Ser Phe Tyr Gly Asn Ser Ile
1               5                   10

<210> 148
<211> 9
<212> PRT
<213> Artificial Sequence

<220>
<223> Description of Artificial Sequence: peptide

<220>
<221> PEPTIDE
<222> (1)..(9)
<223> /note="Start 93"

<400> 148
His Leu Glu Thr Phe Lys Ala Val Leu
1               5

<210> 149
<211> 9
<212> PRT
<213> Artificial Sequence

<220>
<223> Description of Artificial Sequence: peptide

<220>
<221> PEPTIDE
<222> (1)..(9)
<223> /note="Start 319"

<400> 149
His Val Met Asn Pro Leu Glu Thr Leu
1               5

<210> 150
<211> 10
<212> PRT
<213> Artificial Sequence

<220>
<223> Description of Artificial Sequence: peptide

<220>
<221> PEPTIDE
<222> (1)..(10)
<223> /note="Start 18"

<400> 150
Ser Val Trp Thr Ser Pro Arg Arg Leu Val
1               5                   10

<210> 151
<211> 10
<212> PRT
<213> Artificial Sequence

<220>
<223> Description of Artificial Sequence: peptide

<220>
<221> PEPTIDE
<222> (1)..(10)
<223> /note="Start 20"

<400> 151
Trp Thr Ser Pro Arg Arg Leu Val Glu Leu
1               5                   10

<210> 152
<211> 9
<212> PRT
<213> Artificial Sequence

<220>
<223> Description of Artificial Sequence: peptide

<220>
<221> PEPTIDE
<222> (1)..(9)
<223> /note="Start 26"

<400> 152
Leu Val Glu Leu Ala Gly Gln Ser Leu
1               5

<210> 153
<211> 9

<212> PRT
<213> Artificial Sequence

<220>
<223> Description of Artificial Sequence: peptide

<220>
<221> PEPTIDE
<222> (1)..(9)
<223> /note="Start 51"

<400> 153
Glu Leu Phe Pro Pro Leu Phe Met Ala
1               5

<210> 154
<211> 9
<212> PRT
<213> Artificial Sequence

<220>
<223> Description of Artificial Sequence: peptide

<220>
<221> PEPTIDE
<222> (1)..(9)
<223> /note="Start 67"

<400> 154
Gln Thr Leu Lys Ala Met Val Gln Ala
1               5

<210> 155
<211> 10
<212> PRT
<213> Artificial Sequence

<220>
<223> Description of Artificial Sequence: peptide

<220>
<221> PEPTIDE
<222> (1)..(10)
<223> /note="Start 70"

<400> 155
Lys Ala Met Val Gln Ala Trp Pro Phe Thr
1               5                   10

<210> 156
<211> 9
<212> PRT
<213> Artificial Sequence

<220>
<223> Description of Artificial Sequence: peptide

<220>
<221> PEPTIDE
<222> (1)..(9)
<223> /note="Start 78"

<400> 156
Phe Thr Cys Leu Pro Leu Gly Val Leu
1               5

<210> 157
<211> 9
<212> PRT
<213> Artificial Sequence

<220>
<223> Description of Artificial Sequence: peptide

<220>
<221> PEPTIDE
<222> (1)..(9)
<223> /note="Start 84"

<400> 157
Gly Val Leu Met Lys Gly Gln His Leu
1               5

<210> 158
<211> 10
<212> PRT
<213> Artificial Sequence

<220>
<223> Description of Artificial Sequence: peptide

<220>
<221> PEPTIDE
<222> (1)..(10)

<223> /note="Start 95"

<400> 158
Glu Thr Phe Lys Ala Val Leu Asp Gly Leu
1               5                   10

<210> 159
<211> 9
<212> PRT
<213> Artificial Sequence

<220>
<223> Description of Artificial Sequence: peptide

<220>
<221> PEPTIDE
<222> (1)..(9)
<223> /note="Start 133"

<400> 159
Trp Thr Val Trp Ser Gly Asn Arg Ala
1               5

<210> 160
<211> 10
<212> PRT
<213> Artificial Sequence

<220>
<223> Description of Artificial Sequence: peptide

<220>
<221> PEPTIDE
<222> (1)..(10)
<223> /note="Start 155"

<400> 160
Met Thr Lys Lys Arg Lys Val Asp Gly Leu
1               5                   10

<210> 161
<211> 9
<212> PRT
<213> Artificial Sequence

<220>

<223> Description of Artificial Sequence: peptide

<220>
<221> PEPTIDE
<222> (1)..(9)
<223> /note="Start 165"

<400> 161
Ser Thr Glu Ala Glu Gln Pro Phe Ile
1               5

<210> 162
<211> 9
<212> PRT
<213> Artificial Sequence

<220>
<223> Description of Artificial Sequence: peptide

<220>
<221> PEPTIDE
<222> (1)..(9)
<223> /note="Start 180"

<400> 162
Asp Leu Phe Leu Lys Glu Gly Ala Cys
1               5

<210> 163
<211> 9
<212> PRT
<213> Artificial Sequence

<220>
<223> Description of Artificial Sequence: peptide

<220>
<221> PEPTIDE
<222> (1)..(9)
<223> /note="Start 198"

<400> 163
Lys Val Lys Arg Lys Lys Asn Val Leu
1               5

<210> 164

<211> 9
<212> PRT
<213> Artificial Sequence

<220>
<223> Description of Artificial Sequence: peptide

<220>
<221> PEPTIDE
<222> (1)..(9)
<223> /note="Start 205"

<400> 164
Val Leu Arg Leu Cys Cys Lys Lys Leu
1               5

<210> 165
<211> 9
<212> PRT
<213> Artificial Sequence

<220>
<223> Description of Artificial Sequence: peptide

<220>
<221> PEPTIDE
<222> (1)..(9)
<223> /note="Start 222"

<400> 165
Asp Ile Lys Met Ile Leu Lys Met Val
1               5

<210> 166
<211> 9
<212> PRT
<213> Artificial Sequence

<220>
<223> Description of Artificial Sequence: peptide

<220>
<221> PEPTIDE
<222> (1)..(9)
<223> /note="Start 224"

<400> 166
Lys Met Ile Leu Lys Met Val Gln Leu

1            5

<210> 167
<211> 9
<212> PRT
<213> Artificial Sequence

<220>
<223> Description of Artificial Sequence: peptide

<220>
<221> PEPTIDE
<222> (1)..(9)
<223> /note="Start 234"

<400> 167
Ser Ile Glu Asp Leu Glu Val Thr Cys
 1            5

<210> 168
<211> 10
<212> PRT
<213> Artificial Sequence

<220>
<223> Description of Artificial Sequence: peptide

<220>
<221> PEPTIDE
<222> (1)..(10)
<223> /note="Start 234"

<400> 168
Ser Ile Glu Asp Leu Glu Val Thr Cys Thr
 1            5                   10

<210> 169
<211> 10
<212> PRT
<213> Artificial Sequence

<220>
<223> Description of Artificial Sequence: peptide

<220>
<221> PEPTIDE

<222> (1)..(10)
<223> /note="Start 237"

<400> 169
Asp Leu Glu Val Thr Cys Thr Trp Lys Leu
1               5                   10

<210> 170
<211> 9
<212> PRT
<213> Artificial Sequence

<220>
<223> Description of Artificial Sequence: peptide

<220>
<221> PEPTIDE
<222> (1)..(9)
<223> /note="Start 240"

<400> 170
Val Thr Cys Thr Trp Lys Leu Pro Thr
1               5

<210> 171
<211> 10
<212> PRT
<213> Artificial Sequence

<220>
<223> Description of Artificial Sequence: peptide

<220>
<221> PEPTIDE
<222> (1)..(10)
<223> /note="Start 261"

<400> 171
Asn Leu Arg Arg Leu Leu Leu Ser His Ile
1               5                   10

<210> 172
<211> 10
<212> PRT
<213> Artificial Sequence

<220>
<223> Description of Artificial Sequence: peptide

<220>
<221> PEPTIDE
<222> (1)..(10)
<223> /note="Start 325"

<400> 172
Glu Thr Leu Ser Ile Thr Asn Cys Arg Leu
1               5                   10

<210> 173
<211> 9
<212> PRT
<213> Artificial Sequence

<220>
<223> Description of Artificial Sequence: peptide

<220>
<221> PEPTIDE
<222> (1)..(9)
<223> /note="Start 368"

<400> 173
Pro Leu Gln Ala Leu Leu Glu Arg Ala
1               5

<210> 174
<211> 9
<212> PRT
<213> Artificial Sequence

<220>
<223> Description of Artificial Sequence: peptide

<220>
<221> PEPTIDE
<222> (1)..(9)
<223> /note="Start 382"

<400> 174
Asp Leu Val Phe Asp Glu Cys Gly Ile
1               5

<210> 175
<211> 10
<212> PRT
<213> Artificial Sequence

<220>
<223> Description of Artificial Sequence: peptide

<220>
<221> PEPTIDE
<222> (1)..(10)
<223> /note="Start 382"

<400> 175
Asp Leu Val Phe Asp Glu Cys Gly Ile Thr
1               5                   10

<210> 176
<211> 9
<212> PRT
<213> Artificial Sequence

<220>
<223> Description of Artificial Sequence: peptide

<220>
<221> PEPTIDE
<222> (1)..(9)
<223> /note="Start 383"

<400> 176
Leu Val Phe Asp Glu Cys Gly Ile Thr
1               5

<210> 177
<211> 9
<212> PRT
<213> Artificial Sequence

<220>
<223> Description of Artificial Sequence: peptide

<220>
<221> PEPTIDE
<222> (1)..(9)
<223> /note="Start 389"

<400> 177

Gly Ile Thr Asp Asp Gln Leu Leu Ala
1               5

<210> 178
<211> 10
<212> PRT
<213> Artificial Sequence

<220>
<223> Description of Artificial Sequence: peptide

<220>
<221> PEPTIDE
<222> (1)..(10)
<223> /note="Start 401"

<400> 178
Ser Leu Ser His Cys Ser Gln Leu Thr Thr
1               5                   10

<210> 179
<211> 10
<212> PRT
<213> Artificial Sequence

<220>
<223> Description of Artificial Sequence: peptide

<220>
<221> PEPTIDE
<222> (1)..(10)
<223> /note="Start 473"

<400> 179
Glu Leu Gly Arg Pro Ser Met Val Trp Leu
1               5                   10

<210> 180
<211> 10
<212> PRT
<213> Artificial Sequence

<220>
<223> Description of Artificial Sequence: peptide

<220>

<221> PEPTIDE
<222> (1)..(10)
<223> /note="Start 481"

<400> 180
Trp Leu Ser Ala Asn Pro Cys Pro His Cys
1               5                   10

<210> 181
<211> 10
<212> PRT
<213> Artificial Sequence

<220>
<223> Description of Artificial Sequence: peptide

<220>
<221> PEPTIDE
<222> (1)..(10)
<223> /note="Start 493"

<400> 181
Arg Thr Phe Tyr Asp Pro Glu Pro Ile Leu
1               5                   10

<210> 182
<211> 27
<212> PRT
<213> Artificial Sequence

<220>
<223> Description of Artificial Sequence: HLA-A*0201
      binding peptide

<400> 182
Ser Gln Phe Leu Ser Leu Gln Cys Leu Gln Ala Leu Tyr Val Asp Ser
1               5                   10                  15

Leu Phe Phe Leu Arg Gly Arg Leu Asp Gln Leu
            20                  25

<210> 183
<211> 27
<212> PRT
<213> Artificial Sequence

<220>
<223> Description of Artificial Sequence: HLA-A*0201
      binding peptide

<400> 183
Gly Asn Ser Ile Ser Ile Ser Ala Leu Gln Ser Leu Leu Gln His Leu
1               5                   10                  15

Ile Gly Leu Ser Asn Leu Thr His Val Leu Tyr
            20                  25

<210> 184
<211> 27
<212> PRT
<213> Artificial Sequence

<220>
<223> Description of Artificial Sequence: HLA-A*0201
      binding peptide

<400> 184
Lys Ala Val Leu Asp Gly Leu Asp Val Leu Leu Ala Gln Glu Val Arg
1               5                   10                  15

Pro Arg Arg Trp Lys Leu Gln Val Leu Asp Leu
            20                  25

<210> 185
<211> 27
<212> PRT
<213> Artificial Sequence

<220>
<223> Description of Artificial Sequence: HLA-A*0201
      binding peptide

<400> 185
Val Met Leu Thr Asp Val Ser Pro Glu Pro Leu Gln Ala Leu Leu Glu
1               5                   10                  15

Arg Ala Ser Ala Thr Leu Gln Asp Leu Val Phe
            20                  25

<210> 186
<211> 27
<212> PRT

<213> Artificial Sequence

<220>
<223> Description of Artificial Sequence: peptide

<400> 186
Trp Thr Val Trp Ser Gly Asn Arg Ala Ser Leu Tyr Ser Phe Pro Glu
1               5                   10                  15

Pro Glu Ala Ala Gln Pro Met Thr Lys Lys Arg
            20                  25

<210> 187
<211> 27
<212> PRT
<213> Artificial Sequence

<220>
<223> Description of Artificial Sequence: peptide

<400> 187
Asp Glu Ala Leu Ala Ile Ala Ala Leu Glu Leu Leu Pro Arg Glu Leu
1               5                   10                  15

Phe Pro Pro Leu Phe Met Ala Ala Phe Asp Gly
            20                  25

<210> 188
<211> 27
<212> PRT
<213> Artificial Sequence

<220>
<223> Description of Artificial Sequence: peptide

<400> 188
Gln Ser Leu Leu Gln His Leu Ile Gly Leu Ser Asn Leu Thr His Val
1               5                   10                  15

Leu Tyr Pro Val Pro Leu Glu Ser Tyr Glu Asp
            20                  25

<210> 189
<211> 27
<212> PRT
<213> Artificial Sequence

<220>
<223> Description of Artificial Sequence: peptide

<400> 189
Val Phe Asp Glu Cys Gly Ile Thr Asp Asp Gln Leu Leu Ala Leu Leu
1               5                   10                  15

Pro Ser Leu Ser His Cys Ser Gln Leu Thr Thr
            20                  25

<210> 190
<211> 27
<212> PRT
<213> Artificial Sequence

<220>
<223> Description of Artificial Sequence: peptide

<400> 190
Ile Pro Val Glu Val Leu Val Asp Leu Phe Leu Lys Glu Gly Ala Cys
1               5                   10                  15

Asp Glu Leu Phe Ser Tyr Leu Ile Glu Lys Val
            20                  25

<210> 191
<211> 27
<212> PRT
<213> Artificial Sequence

<220>
<223> Description of Artificial Sequence: peptide

<400> 191
Pro Thr Leu Ala Lys Phe Ser Pro Tyr Leu Gly Gln Met Ile Asn Leu
1               5                   10                  15

Arg Arg Leu Leu Leu Ser His Ile His Ala Ser
            20                  25

<210> 192
<211> 27
<212> PRT
<213> Artificial Sequence

<220>
<223> Description of Artificial Sequence: peptide

<400> 192
Phe Leu Lys Glu Gly Ala Cys Asp Glu Leu Phe Ser Tyr Leu Ile Glu
1               5                   10                  15

Lys Val Lys Arg Lys Lys Asn Val Leu Arg Leu
            20                  25

<210> 193
<211> 27
<212> PRT
<213> Artificial Sequence

<220>
<223> Description of Artificial Sequence: peptide

<400> 193
Glu Val Thr Cys Thr Trp Lys Leu Pro Thr Leu Ala Lys Phe Ser Pro
1               5                   10                  15

Tyr Leu Gly Gln Met Ile Asn Leu Arg Arg Leu
            20                  25

<210> 194
<211> 11
<212> PRT
<213> Moloney Murine Leukemia Virus

<220>
<221> PEPTIDE
<222> (1)..(11)
<223> /note="Proteasome fragment"

<400> 194
Pro Ser Ser Ser Trp Asp Phe Ile Thr Val Asp
1               5                   10

<210> 195
<211> 10
<212> PRT
<213> Artificial Sequence

<220>
<223> Description of Artificial Sequence: peptide

<400> 195
Phe Leu Pro Ser Asp Tyr Phe Pro Ser Val
1               5                   10

<210> 196
<211> 10
<212> PRT
<213> Artificial Sequence

<220>
<223> Description of Artificial Sequence: peptide

<400> 196
Phe Leu Pro Ser Asp Cys Phe Pro Ser Val
1               5                   10

<210> 197
<211> 23
<212> DNA
<213> Artificial Sequence

<220>
<223> Description of Artificial Sequence: OPC 189

<220>
<221> misc_feature
<222> (1)..(23)

<400> 197
ctgtactcat ttccagagcc aga                                23

<210> 198
<211> 24
<212> DNA
<213> Artificial Sequence

<220>
<223> Description of Artificial Sequence: OPC 190

<220>
<221> misc_feature
<222> (1)..(24)

<400> 198
tattgagagg gtttccaagg ggtt                               24

<210> 199
<211> 9
<212> PRT
<213> Artificial Sequence

<220>
<223> Description of Artificial Sequence: peptide

<400> 199
Leu Tyr Val Asp Ser Leu Phe Phe Leu
1               5

<210> 200
<211> 12
<212> PRT
<213> Artificial Sequence

<220>
<223> Description of Artificial Sequence: peptide

<400> 200
Leu Gln Ala Leu Tyr Val Asp Ser Leu Phe Phe Leu
1               5                   10

<210> 201
<211> 11
<212> PRT
<213> Artificial Sequence

<220>
<223> Description of Artificial Sequence: peptide

<400> 201
Gln Ala Leu Tyr Val Asp Ser Leu Phe Phe Leu
1               5                   10

<210> 202
<211> 10
<212> PRT
<213> Artificial Sequence

<220>
<223> Description of Artificial Sequence: peptide

<400> 202

Gln Ser Leu Leu Gln His Leu Ile Gly Leu
1               5                   10

## Description

### §1 Field of the invention

The present invention relates to the field of molecular biology and immunology. In particular it relates to vaccines and methods for providing vaccines which elicit immune responses when administered to a mammal, in particular a human. The preferred elicited immune response is a T cell response, elicited by peptide T cell epitopes. These vaccines find their application in many fields ranging from cancer treatments to treatments or prophylaxis of infectious diseases such as Aids.

The present invention provides novel methods for selecting the peptide sequences from an intact antigen which will lead to a proper (T cell) immune response upon administration in a suitable vehicle. The epitopes and vaccines are, of course, also part of the present invention. It is by now well known that peptides which are to be presented to T cell receptors need to fulfil a number of requirements. For different haplotypes different anchor residues are required; only peptides of a certain length can be presented, specific cleavage sites must be present around the peptide, signals to transport the peptide to the surface in the right context must be present, the stability of the bond between peptide and presenting molecule is relevant, etc. All these requirements have been used by the present group to design or localise peptides that are good T cell epitopes and are thus suitable peptides for vaccination. The present invention provides a further improvement in the selection (and thus the production) of T cell epitopes, especially in relation with their cleavage. The present invention relates to the cleavage of the C-terminal end of T cell epitopes. The present invention discloses that the 20S proteasome is involved in the mentioned cleavage. Proteasomes play a pivotal role in the generation of antigenic peptides (Coux et al., 1996; Rock et al., 1999) .

The proteasome is a multicatalytic protease complex (Brown et al., 1991; Ortiz-Navarrette et al., 1991), localized in the cytoplasm and/or the nucleus (Rivett 1993). The proteasome is a 2000-kDa particle, also known as the 26S proteasome, with a catalytic core known as the 20S proteasome (Hershko and Ciechanover, 1992; Rechsteiner et al., 1993; Lîwe et al., 1995). The 26S proteasome can cleave folded and unfolded proteins in an ATP dependent pathway, through both ubiquitin-dependent and ubiquitin-independent pathways (Hershko and Ciechanover, 1992; Murakami et al., 1992; review Gerards et al., 1998). In vitro, 20S proteasomes only degrade fully unfolded proteins or synthetic peptides not longer than approx. 40 residues.

The eukaryotic 20S proteasome comprises four rings, stacked in a rod-like shape. Each ring contains seven different subunits. The outer two rings are made of α-subunits, the inner two rings of β-subunits. Only three of the β-subunits have catalytic activity. Eukaryotic proteasomes have several distinct proteolytic cleavage specificities. The specificities are designated chymotrypsin-like, trypsin-like, peptidylglutamyl-like, branched and small-neutral peptide hydrolyzing (Leibovitz et al., 1995; Cardozo et al., 1996). Two types of proteasomes can be discriminated, namely constitutive (or household) and the IFN-γ dependent immuno proteasomes. In the constitutive proteasome, the three catalytic β-subunits are called X, Y, and Z. In the immuno proteasomes, the three IFN-γ inducible subunits are called LMP2, LMP7, and MECL-1 (Gaczynska et al., 1993; Boes et al., 1994; Griffin et al., 1998).

Virtually all currently available vaccines are not rationally designed in the sense of detailed knowledge of minimal essential epitopes and the rules of antigen processing and presentation. Rather, the available vaccines are based on empirical knowledge of protection. A major objective of the present invention is to develop a new generation of more rationally designed vaccines which are effective, safe, easy to manufacture and standardise, stable, inexpensive and associated with long lasting protection. In the present invention we disclose the use of proteasome digestion in identifying T cell epitopes.

Thus the invention provides a method for selecting and/or producing a T cell epitope, comprising subjecting a precursor peptide or polypeptide to the action of a 20S proteasome or a functional equivalent thereof to determine the location of the c-terminus of said T cell epitope. It has been found that by adding this test to the already known criteria for selecting T cell epitopes it is now possible to predict with high likelihood of success which T cell epitopes are suitable vaccine candidates. That the C-terminus is determined by proteasome action is clear from our finding (in press) that the substitution of the residue at the C-terminus or flanking the C-terminus with another residue results in barogation of proteasome processing and in the T cell epitope not being produced.

A precursor peptide or polypeptide may be any (poly)peptide suspected to contain T cell epitopes. It is preferred to carry out a preselection using other known characteristics of T cell epitopes before designing or isolating pecursors to be tested for proteasome cleavage. The test does not have to be a real physical test. Once the action of the proteasome is well understood, the test may be carried out looking for typical sites to be present after the action of the (immune-related) proteasome in the same manner as is now common for anchor sites.

It is presently believed that the C-terminus of the T cell epitope is determined by proteasome action. In order to obtain the right N-terminus the action of (ER-restricted) proteases may be required. Of course the combined action of proteasome and the proteases mentioned above is preferred according to the present invention. As already stated it is also preferred to test the precursors for other T cell epitope characteristics, be it physically, or by computer, be it before or after the (physical) action of the proteasome. Thus in another embodiment the invention comprises a method for selecting and/or producing T cell epitopes wherein said precursor peptide or polypeptide is tested for other T cell epitope characteristics, in particular testing for the right anchor residues, the proper cleavage sites, the proper pathway signals, and/or the stability of the MHC-T cell epitope complex.

Together with these methods developed also by the group of present inventors a T cell epitope can be predicted, selected and/or produced with a high degree of certainty. The selection of peptide epitopes for a given combination of antigen and HLA class I molecule according to the invention may be e.g. divided in the following steps:
1. Computer prediction of peptides within the primary sequence of the antigen that are most likely to bind to the HLA class I molecule concerned, by comparison with the relevant motif for MHC class I binding.
2. Measurement of the actual binding of the selected peptides to the MHC molecule concerned using assays that determine HLA-peptide binding and stability of the HLA-peptide complex herein).
3. Screening of the peptides (selected by steps 1 & 2) and their flanking sequences (in the context of the intact antigen) for compliance with the rules for proteasome cleavage of natural protein sequences
4. Screening of the peptides (selected by steps 1 & 2) and their flanking sequences (in the context of the intact antigen) for compliance with the rules for effective peptide transport and loading into HLA class I molecules.

The selected T cell epitopes are also part of the present invention, as are epitopes derived therefrom which are improved by site-directed mutation shuffling techniques and library based techniques (such as phage display). Thus the invention also provides in another embodiment a T cell epitope obtainable by a method as described above. Selected peptide epitopes (see steps 1-4) are incorporated into the following prototype vaccines, the efficacy of which is compared in the appropriate HLA transgenic mouse model:
i. Mixture of synthetic peptides in adjuvants.
ii. Mixture of synthetic peptides loaded onto dendritic cells.
iii. Recombinant protein, synthesized in - and purified from - E.coli, consisting of a string bead arrangement of peptide-epitopes which are separated from each other by proteolytic cleavage sites.
   Protein administered in adjuvants.
iv. Recombinant protein (see iii; mannosylated) loaded onto dendritic cells.
v. Recombinant DNA construct (naked DNA) that encodes string-beads of peptide epitopes which are separated by proteolytic cleavage sites.
vi. Recombinant Canary pox virus that encodes string- beads of peptide epitopes which are separated by proteolytic cleavage sites.
vii. Recombinant human adenovirus that encodes string- beads of peptide epitopes which are separated by proteolytic cleavage sites.

Efficacy of the various vaccination protocols is assayed by restimulation in mixed lymphocyte cultures of spleen cells of the immunized animals with e.g. autologous LPS B-cell blasts that are loaded with the relevant peptide(s), followed by measurement of the reactivity of the resulting'T cell cultures against target cells that either present synthetic peptides or the naturally processed epitopes.

The peptide epitopes are also used for the induction of antigen-specific T cell activity in HLA-transgenic mixed lymphocyte cultures in vitro. To that end, the peptide(s) of choice are loaded onto either syngeneic LPS B-cell blasts or dendritic cells. These cells are irradiated and used as stimulator cells with nylonwool passed spleen cells of HLA-transgenic mice. After in vitro stimulation for one or two weeks, the reactivity of the resulting T cell populations can be measured against target cells that either present synthetic peptides or the naturally processed epitopes.

Rational design of vaccines has clear advantages. Safety is one. For example DNA or viral vector vaccines for HPV16 E6 and E7 are intrinsically unsafe if such vaccines contain functional oncogenes, but safe if the DNA or viral vector encodes string beads of epitopes, a preferred embodiment of the present invention. Additional advantages are effectiveness and simplicity. Only effectively immunizing components are included. Irrelevant sequences are deleted, easing manufacture and standardization, enhancing stability and decreasing cost.

The design of effective T cell epitope vaccines hinges on the accurate selection of immunogenic peptides. By means of the current invention. we have considerably improved the selection procedure. In a number of embodiments of the present invention vaccines are made in situ by providing cells with a nucleic acid encoding at least one of the T cell epitopes according to the invention. Thus these nucleic acids are also part of the invention. In this embodiment the invention provides a nucleic acid encoding at least one T cell epitope selected according to a method as disclosed above. Preferred nucleic acids are those that comprise at least two sequences encoding a T cell epitope, separated by a sequence encoding at least one protelytic cleavage site, especially when incorporated in a gene delivery vehicle, such as an adenovirus.

Thus in another embodiment the invention provides a gene delivery vehicle comprising a nucleic acid as disclosed above. This important embodiment of the present invention provides e.g. a method which induces T cell reactivity against multiple pre-selected T cell epitopes by immunization with a recombinant adenovirus (rAd) vector that contains multipe T cell epitopes in a string-of-bead fashion in which the T cell epitopes are linked to each other by proteolytic cleavage sites. The linkage of T cell epitopes by spacer sequences ensures that the T cell epitopes are efficiently processed and presented to T cells. Therefore, the incorporation of multiple T cell epitopes spaced by linker-sequences preferably into recombinant adenovectors represents an important and powerful new approach for the induction of strong antiviral and anti-tumor T cell immunity that is directed against multiple T cell targets.

Other gene delivery vehicles are by now known to the person skilled in the art an can be applied using his general knowledge. Also other ways of administering epitopes according to the invention for eliciting an immune response are provided by the present invention. Peptide vaccine technology has been described extensively and the possibilities are well known. Adjuvantia, routes of administration and other parameters therefor need no further description here.

Thus the invention provides a pharmaceutical composition comprising a gene delivery vehicle as described herein or otherwise known in the art, encoding at least one epitope according to the invention, as well as a pharmaceutical composition comprising at least one epitope according to the invention and/or a pharmaceutical composition comprising a proteinaceous molecule obtainable by expression of a nucleic acid according to the invention.

Typically a pharmaceutical composition to be used as a vaccine may comprise an adjuvant. Because the present invention mainly relates to peptides and their use in vaccine in any way, peptide presentation in the right context may, in some embodiments where the subject to be treated does not have sufficient capability for presenting peptides, be advantageous. Also this presentation may be useful for preventing breakdown or enhancing presentation.

Thus the invention also provides a pharmaceutical composition comprising an antigen presenting moiety, in particular a major histocompatibility molecule, especially when present on an antigen presenting cell, such as a dendritic cell.

### Detailed description.

In this study, 20S proteasomes were isolated and purified from the mouse lymphoma cell line that mainly contains immunoproteasomes. Fourteen synthetic peptides with well known CTL-epitopes, and naturally flanking amino acids, were used. After proteasome digestion, peptide fragments were analyzed by electrospray mass spectrometry. In most cases, the C-terminal residue of the most abundant fragments was the same residue found in the CTL-epitope.

This study shows that the C-terminal cleavage site is determined by the proteasome, but that the precise MHC presented CTL-epitope is generally not generated by the proteasome. Most peptide fragments with the correct C-terminal residue had several additional amino acids at the N-terminus. The optimal MHC class I presentable peptide length is most likely generated by N-terminal trimming by ER restricted peptidases.

### Experimental procedures

### Peptides

Peptides were synthesized on a multiple peptide synthesizer (Abimed AMS 422) as described (Gausepohl et al., 1990). Peptides were purified by RP-HPLC in an acetonitrile-water gradient and lyophilized overnight.

### Proteasome digestion assay

20S proteasomes were purified from a mouse lymphoma cell line that mainly contains immunoproteasomes after stimulation with IFN-γ as described (Groettrup et al., 1995) .

Peptides (26 mers, 20 µg) were incubated with 1 µg of purified proteasome at 37°C for 1, 4 and 24 hours in 300 µl of proteasome digestion buffer (PDB) as described (Eggers et al., 1995). Addition of 30 µl of trifluoroacetic acid stopped the digestion. Before analysis by electrospray mass spectrometry, peptide digests were kept at -70°C.

### Electrospray Mass spectrometry

Electrospray ionization mass spectrometry was performed on a hybrid quadrupole time-of-flight mass spectrometer, a Q-TOF (Micromass, Manchester, UK), equipped with an on-line nanoelectrospray interface (capillary tip 20 µm internal diameter ^{*} 90 µm outer diameter) with an approximate flow rate of 250 nl/min. This flow was obtained by splitting of the 0.4 ml/min flow of a conventional high pressure gradient system 1 to 1000, using an Accurate flow splitter (LC Packings, Amsterdam, The Netherlands).

Injections were done with a dedicated micro/nano HPLC autosampler, the FAMOS (LC Packings, Amsterdam, The Netherlands), equipped with two extra valves for phase system switching experiments. Digestion solutions were diluted five times in water/methanol/acetic acid 95/5/1 v/v/v, and 1 µl was trapped on the precolumn (MCA-300-05-C8; LC Packings, Amsterdam, The Netherlands) in water/methanol/acetic acid 95/5/1/ v/v/v. Washing of the precolumn was done for three minutes to remove the buffers present in the digests. Subsequently, the trapped analytes were eluted with a steep gradient going from 70% B to 90% B in 10 minutes, with a flow of 250 nl/min. (A: water/methanol/acetic acid 95/5/1 v/v/v; B water/methanol/acetic acid 10/90/1 v/v/v.) This low elution rate allows for a few additional MS/MS experiments if necessary during the same elution.

Mass spectra were recorded from mass 50-2000 Da every second with a resolution of 5000 FWHM. The resolution allows direct determination of the monoisotopic mass, also from multiple charged ions. In MS/MS mode ions were selected with a window of 2 Da with the first quadrupole and fragments were collected with high efficiency with the orthogonal time-of-flight mass spectrometer. The collision gas applied was argon (4^{*}10⁻⁵ mbar), and the collision voltage appr. 30V.

The peaks in the mass spectra were searched in the digested precursor peptide using the Biolynx/proteins software supplied with the mass spectrometer.

### TNF release assay

Measurement of secreted TNF-γ by stimulated CTL's was performed with a bioassay, using WEHI 164 clone 13 cells, as described previously (Traversari et al. 1992). Percentage TNF- release of triplicate wells was calculated as follows: % TNF release = [(A550-560nm experimental wells - A550-650 nm wells containing medium only)/A550-650 nm wells containing 500 pg/ml TNF -A550-650 nm wells containing medium only)].

### Results

Highly purified synthetic peptides (26 residues) were digested by the 20S proteasome at 37°C at 3 time intervals (1 hr, 4 hrs and 24 hrs). After digestion, the digest mixtures were analyzed by electrospray mass spectrometry for the resulting peptide fragments.

Table 1 shows the sequence of the fourteen synthetic peptide substrates used for this study. All peptides contain well-defined CTL-epitopes of murine origin with their naturally flanking amino acids.

We systematically investigated the specificity of the 20S proteasome, isolated from mouse lymphoma cells, on these substrates. In 12 out of fourteen peptide substrates, the correct C-terminal residue was generated by the 20S proteasome (see table 1). There is a high degree of preference by the 20S proteasome for cleavage after the C-terminal residue of the CTL-epitope, but cleavage by the 20S proteasome, apparently, does not always generate the exact CTL-epitope. For instance, a synthetic peptide derived from Sendai is preferentially cleaved after the C-terminal leucine of the CTL-epitope, but the exact CTL-epitope is not generated (see table 2). In this case, the most prominent epitope-containing fragment is the N-terminally elongated 11-mer GEFAPGNYPAL. This fragment is already generated after 1 hour of digestion, suggesting that this is the important precursor peptide of which the epitope is processed. It was already found in a previous study (Neisig et al., 1995) that the minimal 9-mer epitope FAPGNYPAL is not efficiently translocated by TAP into the endoplasmic reticulum (ER), most likely due to a proline at position 3 of the epitope. In that same study, it was shown that synthetic peptides elongated with natural amino acids were efficiently translocated by TAP. In fact, the most efficiently translocated length variant of the Sendai epitope was the 11-mer GEFAPGNYPAL, also generated by the 20S proteasome.

These findings were similar to our results obtained by proteasome-mediated digestion experiments using the MCF-MuLV peptide substrate harboring the CTL-epitope KSPWFTTL. We found that the 10-mer FNKSPWFTTL and the 11-mer FNKSPWFTTLI were generated by the proteasome (see table 2 and Ossendorp et al., 1996), and were efficiently translocated by TAP into the ER. The exact CTL-epitope KSPWFTTL was not translocated by TAP at all (Neisig et al., 1995).

To investigate the influence of flanking residues upon the cleavage specivicity of the 20S proteasome, three synthetic peptides containing natural variants of a subdominant MuLV were used (see table 3A). The epitope was defined for Moloney MuLV to be located in env-gp70 (SSWDFITV; Sijts et al., 1994). This epitope is identical in the closely related Rauscher MuLV, but is different in only one residue in AKV and Friend MuLV (SSWDYITV).

Strikingly, despite the fact that both peptides have good binding properties to the MHC class I Kb molecule, CTL specific for the Moloney epitope SSWDFITV were not able to recognize Friend MuLV induced FBL-3 tumor cells (data not shown). We have investigated in vitro processing by the 20S proteasome of these peptide substrates containing the natural flanking residues. Besides to the F to Y difference, one other distinct amino acid difference occurred and is located after the valine (V) residue in all three sequences. For the Moloney MuLV peptide substrate we found preferential cleavage after the C-terminal V. The 10-mer PSSSWDFITV is probably the most important precursor epitope peptide. The AKV MuLV peptide substrate containing an Y instead of an F, also shows preferential C-terminal cleavage after valine and no cleavage after tyrosine. The Friend MuLV peptide substrate showed no cleavage after valine, but dominantly after aspartic acid (D). Again, no cleavage has been found after tyrosine.

To investigate the influence of tyrosine (Y) versus phenylalanine (F) on the C-terminal cleavage by the 20S proteasome, 4 analogue synthetic peptide substrates of MuLV were synthesized (see table 3B) . The results in table 3A and table 3B show that there is no difference in cleavage products for the C-terminal residue with regard to the substrates 5A, 5D and 5F (cleavage after V), and the substrates 5C, 5E and 5G (cleavage after D). In the substrates 5C, 5E and 5G, we found that the aspartic acid residue next to the valine most dominantly influenced the C-terminal cleavage site, because no cleavage after valine was found. We also found no influence of the residues F and Y on C-terminal cleavage preferences by the proteasome. From table 2 we can conclude that 20S proteasomes can generate the peptide fragment PSSSWDFITV that should be N-terminally trimmed in the ER to SSWDFITV. It was also found that FBL-3 cells generate the peptide fragment PSSSWDFITVD. This additional aspartate residue at the C-terminus completely abolishes TAP translocation and binding to the MHC class I Kb molecule (data not shown).

These findings can explain the lack of recognition of the Friend MuLV induced tumor cell line FBL-3 by the specific CTL clone 10B6. Synthetic peptide binding experiments were performed with all relevant peptide fragments generated after proteasome-mediated cleavage (results not shown). Peptide SSWDFITV, being the CTL-epitope, was used as a control and was only generated in very low amounts after digestion of peptide substrate 5F.

The CTL-epitope SSWDFITV is the best binding peptide for the MHC class I Kb molecule. A precursor peptide fragment PSSSWDFITV also binds to the MHC class I Kb molecule, but with much lower affinity. The smaller peptide SWDFITV, generated in high amounts by the proteasome, does not detectably bind to the MHC class I Kb molecule. This peptide of 7 amino acids is probably too small for proper binding in the MHC class-I groove. The peptides that have an aspartic acid as C-terminal residue do not bind to the MHC class-I Kb molecule, due to the fact that this aspartic acid residue completely disturbs the binding of the C-terminal anchor residue V into the pocket of the Kb groove.

According to the present invention a number of HLA-A 0201 restricted eptiopes of a tumor associate antigen were identified. The detailed description of their identification is presented below.

The tumor-associated antigen PRAME is highly expressed in a broad array of solid tumors and 30% of acute leukemias, whereas normal tissue expression is confined to the testis, endometrium and at very low levels in ovaries and adrenals. We now report the identification of four HLA-A^{*}0201 presented CTL epitopes in PRAME utilizing the 'reverse immunology' strategy of in vitro sensitization of CTL against predicted epitopes. Next to motif based HLA-A^{*}0201 binding prediction and actual binding assays, analysis of in vitro proteasome mediated digestions of 27-mer polypeptides encompassing candidate epitopes were incorporated in the epitope prediction procedure.

We show that proteasome cleavage pattern analysis, and in particular determination of correct C-terminal cleavage of the putative epitope, is applicable for more selective and accurate prediction of CTL epitopes. Our approach proved more efficacious than previous protocols, avoiding laborious CTL response inductions against peptides that are, despite their high binding affinity for HLA I, not endogenously generated in the processing pathway. The in vitro CTL induction protocol that we used is developed from two protocols known in literature and is an improvement in that it utilizes efficiently all antigen presenting cells present in PBMC. CTL clones recognizing the identified epitopes specifically lyse melanoma, renal cell carcinoma and lung carcinoma cell lines. This shows that these epitopes are expressed on a diversity of tumorcells, making them attractive targets for an immunotherapy against cancer.

### Materials and Methods

### Cell lines

The EBV-transformed B cell line JY (HLA type: A^{*}0201, B7, Cw7, DR4, DRw6, DPw2) was cultured in complete culture medium consisting of Iscove's Modified Dulbecco's Medium (IMDM) (Biowithaker) supplemented with 8% foetal calf serum (FCS) (Hyclone Laboratories Inc., Logan), 100 IU/ml penicillin (Brocades, Pharma, Leiderdorp, The Netherlands) and L-glutamine (2 mM, ICN Biochemicals, Costa Mesa, CA).

Melanoma cell lines Me1603, M453 and FM3 and renal cell carcinoma cell lines MZ1851, MZ1774 and MZ1257 were kindly provided by Dr. P Schrier (Leiden University Medical Center, The Netherlands). Lung carcinoma cell lines GLC-02 and GLC-36 were provided by Dr. L. de Leij (University of Groningen, The Netherlands).

### Peptides

Peptides were synthesized by solid phase strategies on an automated multiple peptide synthesizer (Abimed AMS 422, Langefeld, Germany) using Fmoc chemistry. Peptides were analyzed by reverse phase HPLC, dissolved in 20 µl DMSO, diluted in 0,9% NaCl to a peptide concentration of 1 mg/ml, and stored at -20°C before usage. The fluorescein (FL)-labeled reference peptide as used in the competition based HLA-A^{*}0201 binding assay was synthesized, labeled and characterized as described earlier.

The sequence of the FL-labeled peptide was FLPSDYFPSV wherein we substituted the tyrosine with a cysteine to tag a fluorescein group to the peptide: FLPSDC(FL)FPSV. The 27-mer polypeptides used for in vitro proteasome digestion were synthesized as described above, purified using preparative reverse phase HPLC and lyophilized overnight. Purity was confirmed by massspectrometry.

### Cellular competition based HLA-A^{*}0201 peptide-binding assay

Affinity of peptides for HLA-A^{*}0201 was analyzed using the homozygous HLA-A^{*}0201+ B-LCL JY as described previously, with minor adaptations. Briefly, naturally bound peptides were stripped from the HLA-A^{*}0201 molecules by exposing the JY cells for 90 seconds to ice-cold citric-acid buffer with a pH of 3.1 (1:1 mixture of 0.263 M citric acid and 0.123 M Na2HPO4). Cells were immediately buffered with cold IMDM containing 2% FCS, washed twice in the same medium and resuspended in 2% FCS/IMDM containing 2 µg/ml human β₂-microglublin (Sigma, St. Louis, MO, USA). Subsequently, the stripped JY cells were plated at 4x10⁴/well in a 96-well V-bottom plate together with 150 nM of a known high affinity HLA-A2^{*}0201 binding fluorescein (FL)-labeled reference peptide (HBV nucleocapsid antigen 18 to 27,) and titrated concentrations of competitor-test-peptide.

After incubation for 24 hr at 40°C, cells were washed three times in PBS containing 1% BSA, fixed with 0.5% paraformaldehyde, and analyzed on FACScan flow cytometer (Becton Dickinson). The % inhibition of fluoresceine labeled reference peptide binding was calculated using the following formula: (1-(MF reference and competitor peptide - MF no reference peptide) / (MF reference peptide - MF no reference peptide)) x 100%. The binding affinity of competitor peptide is expressed as the concentration needed to inhibit 50% binding of the FL-labeled reference peptide (IC50). An IC50 ( 5 µM was considered high affinity binding, 5 µM < IC50 ( 15 µM was considered intermediate binding, 15 µM < IC50 ( 100 µM was judged as low affinity binding and IC50 > 100 µM as not binding.

### Peptide-MHC complex dissociation assay

Binding stability at 37°C of peptides complexed with HLA-A*0201 was measured as previously described. In short, JY cells were treated with10⁻⁴ M emetine (Sigma, St. Louis, MO, USA) for 1 hr at 370C to stop de novo synthesis of MHC class I molecules. Subsequently, endogenous bound peptides in HLA-A^{*}0201 were removed by mild-acid treatment (see before) and reconstituted with the peptide of interest at 200 µM in 2% FCS/IMDM containing 2 µg/ml human ß₂-microglublin (Sigma, St. Louis, MO, USA) for 1.5 h at room temperature. Hereafter, cells were washed twice to remove free peptide and incubated at 37°C for 0 h, 2 h, 4 h and 6 hours. Subsequently, expressed HLA-A*0201-peptide complexes on JY cells were stained using the conformation specific Moab BB7.2 and GAM-Fitc and analyzed on a FACScan flow cytometer.
The fluorescence index (FI) was calculated for each sample as: (MFsample - MFbackground) / MFbackground, where MFbackground is the value without peptide. The percentage of residual HLA-A^{*}0201 molecules was calculated by equating for each peptide, the FI of t = 2 h to 100% and then using the formula: % remaining = (FIt=n / FIt=2) x 100%. Because the dissociation of peptides from MHC is a linear process, the stability of the peptide-MHC complexes was measured as the time required for 50% of the molecules to decay (DT50), starting from t=2.
By linear regression analysis of the sequential measurements plotted against the % remaining HLA-A^{*}0201 molecules, the DT50 was calculated. As positive control the known highly stable CTL epitope HBV core (18-27) was used. In vitro proteasome mediated digestions 20S proteasomes were purified from an EBV transformed B-LCL cell line as described. Peptides (27 mers, 20 µg) were incubated with 1 µg of purified proteasome at 37°C for 1 h, 4 h and 24 hours in 300 µl proteasome digestion buffer as described 38. Trifluoroacetic acid (TFA, 30 µl) was added to stop the digestion and samples were stored at-20°C before massspectrometric analysis.

### Mass spectrometry

Electrospray ionization mass spectrometry was performed on a hybrid quadrupole time-of-flight mass spectromter, a Q-TOF (Micromass, Machester, UK), equipped with an on-line nanoelectrospray interface (capillary tip 20 :m internal diameter ^{*} 90 (m outer diameter) with an approximate flow rate of 250 nL/min. This flow was obtained by splitting of the 0.4 mL/min [mL = ml?!] flow of a conventional high pressure gradient system 1 to 1000, using an Acurate flow splitter [LC Packings, Amsterdam, The Netherlands].
Injections were done with a dedicated micro/nano HPLC autosampler, the FAMOS [LC Packings, Amsterdam, The Netherlands], equipped with two extra valves for phase system switching experiments. Digestion solutions were diluted five times in water/methanol/acetic acid 95/5/1 v/v/v, and 1 (L [L of 1?] was trapped on the precolumn (MCA-300-05-C8; LC Packings, Amsterdam, The Netherlands) in water/methanonl/acetic acid 95/5/1 v/v/v. Washing of the precolumn was done for three minutes to remove the buffers present in the digests.
Subsequently, the trapped analytes were eluted with a steep gradient going from 70% B to 90% B in 10 minutes, with a flow of 250 nL/min (A: water/methanol/acetic acid 95/5/1 v/v/v; B: water/methanol/acetic acid 10/90/1 v/v/v). This low elution rate allows for a few additional MS/MS experiments if necessary during the same elution. Mass spectra were recorded from mass 50-2000 Da every second with a resolution of 5000 FWHM. The resolution allows direct determination of the monoisotopic mass, also from multiple charged ions. In MS/MS mode ions were selected with a window of 2 Da with the first quadrupole and fragments were collected with high efficiency with the orthogonal time-of-flight mass spectrometer. The collision gas applied was argon (4^{*}10⁻⁵ mbar), and the collision voltage appr. 30V.
The peaks in the mass spectrum were searched in the digested precursor peptide using the Biolynx/proteins software supplied with the mass spectrometer.

### RT-PCR assay for PRAME expression

Analysis of PRAME mRNA expression was determined by rtPCR. Total cellular RNA was isolated with Trizol (Gibco, Gaithersburg, MD) according to the manufacturer's procedure. Reverse transcription was performed on 5 µg of total RNA in a reaction volume of 25 µl with 5 µl of 5x reverse transcriptase buffer (Promega), 2.5 ?1 each of 10 mM deoxynucleotides (Amersham), 0.5 µg oligo dT15 (15 subscript) primer, 25 U of RNAsin (Promega) and 15 U AMV reverse transcriptase (Promega) .
The reaction was incubated at 42°C for 60 min, heat inactivated for 10 min at 70°C and diluted 2x with water. For PCR amplification, 1 µl of reverse transcribed cDNA reaction mixture was used as a template. PCR primers used for the analysis of PRAME expression were OPC 189 (sense primer, 5'-CTGTACTCATTTCCAGAGCCAGA-3') and OPC 190 (antisense primer, 5'-TATTGAGAGGGTTTCCAAGGGGTT-3') 30, and PCR conditions were 5 min at 94°C followed by 34 cycles consisting of 30 sec at 94°C, 2 min at 64°C, and 3 min at 72°C.

### In vitro CTL response induction and generation of CTL clones

PBMC of HLA-A^{*}0201+ healthy donors were obtained by Ficoll-Paque method.PBMC were separated in a T cell fraction and a fraction containing mainly B cells and monocytes by sheep red blood cell (SRBC) rosetting. The T cell fraction was cryopreserved. To optimally use all antigen presenting cells (APC) present in PBMC we developed a new culture system. The mixture of monocytes and B cells (approximately 1:1) was cultured in 24 wells plates at a concentration of 1x10⁶ cells/well in complete culture medium containing 800 U/ml GM-CSF, 500 U/ml IL-4 and 500 ng/ml CD40 mAb (Serotec) for 6 days. This culture system had a threefold effect:
i) GM-CSF + IL-4 differentiated monocytes into immature dendritic cells,
ii) IL-4 + CD40 mAb resulted in activation and proliferation of B cells and
iii) CD40 mAb caused maturation of the immature dendritic cells.
At day 3 cytokines and CD40 mAb were refreshed. Facs analysis after 6 days culturing revealed approximately equal levels of activated B cells and mature dendritic cells expressing high levels of CD80 and CD86. To further promote maturation the APC-mix was cultured for an additional 2 days with 0.4 ng/ml LPS, 500 U/ml IFNγ (Boehringer) and 500 ng/ml CD40 mAb. At day 8 the APC-mix was pulsed with 50 µg/ml peptide (each peptide seperately) for 4 h at room temperature (RT) and subsequently irradiated (30 Gy) and washed to remove free peptide. The cryopreserved autologous T cell fraction was thawed and depleted from CD4+ T cells using magnetic beads (Dynal). The primary induction was performed in 96 well U-bottom plates (Costar): APC at a concentration of 10,000/well were co-cultured with 50,000 CD8+ T cells/well in culture medium, containing 10% human pooled serum (HPS), 5 ng/ml IL-7 and 0.1 ng/ml IL-12.
At day 7 after initiation of induction the CTL culture was harvested, washed and restimulated at a concentration of 40,000 responder cells/well of 96-well U-bottom plate in culture medium containing 10% HPS, ng/ml IL-7 and 0.1 ng/ml IL-12.
Autologous activated B cells, irradiated (75 Gy) and peptide pulsed (50 µg/ml) for 4 h at RT in culture medium containing 2% FCS and 3 µg/ml ß₂ microglobulin (Sigma, St. Louis, MO, USA) after mild acid elution to remove naturally presented peptides from the MHC molecules (see material and methods MHC binding assay), were used at a concentration of 10,000 cells/well as restimulator APC. Restimulations were repeated at day 14 and 21 in a similar way, with the exception of IL-7 being replaced by 20 IU/ml 1L-2. At day'29 the CTL bulk culture was cloned by standard limiting dilution procedures. CTL clones were maintained by aspecific stimulation every 7 to 12 days using a feeder mixture consisting of allogeneic PBMC and EBV transformed B cells, in culture medium containing 10% FCS, 1.5% leucoagglutinin and 240 IU/ml IL-2.

### ⁵¹Cr cytotoxicity assay

CTL activity was measured in a standard chromium release assay. Briefly, after ⁵¹Cr labeling, target cells were pulsed with the designated peptide concentrations for 30 min or longer at 37°C. Target cells were added to various number of effector cells in a final volume of 100 µl complete culture medium in 96-well-U-bottom plates. After 4 h incubation at 37°C supernatants were harvested. The mean % specific lysis of triplicate wells was calculated according to: (Experimental release - Spontaneous release) / (Maximal release - Spontaneous release) x 100%.

### Stable transfection with fulllength PRAME encoding plasmid

Full length PRAME cDNA was kindly provided by Dr. P. Coulie (Ludwig Institute for Cancer Research, Brussels, Belgium). The PRAME encoding insert was cloned into vector pDR2 confering hygromycine resistance. The renal-cell carcinoma cell line MZ1851 was transfected with pDR2-PRAME using Fugene (Boehringer) as transfection reagents according to the manufacturer's instruction. After 48 h hygromycine (100 (g/ml) was added to select transfected cells.

### Results

Identification of HLA-A^{*}0201 binding peptides from PRAME To select candidate HLA-A^{*}0201 binding peptides from PRAME the amino acid sequence was screened for HLA-A^{*}0201 binding motif containing peptides using a combination of two known binding prediction algorithms. Only peptides of 9 aa. or 10 aa. length were included, taking into account the low prevalence of HLA-A^{*}0201 restricted CTL epitopes of 8 aa. or 11 aa. length. In total, 128 peptides (65 nonamers and 63 decamers) were synthesized in order to determine their actual binding affinity for HLA-A^{*}0201. A competition-based cellular binding assay was used to determine the concentration of test peptide inhibiting the binding of a fluorescein labeled high affinity binding reference peptide (HBV nucleocapsid 18-27) for 50% (IC50). Thirteen high affinity binding peptides were identified (IC50 ( 5 5 (M), 33 peptides bound with intermediate affinity (5 (M < IC50 ( 15 (M), whereas the other peptides displayed a low affinity (15 (M < IC50 ( 100 (M) or undetectable affinity (IC50 > 100 (M) (table A).

To more precisely define binding characteristics, the dissociation rate of peptides binding with high affinity to HLA-A^{*}0201 and in addition VLDGLDVLL (aa. 100-108) and ALLERASATL (aa. 371-380) complexed with HLA-A^{*}0201 at 37°C was measured to assess peptide-MHC stability under physiologic temperatures. The HLA-A*0201 homozygous B-LCL JY cells were stripped of their endogenously bound MHC class I peptides by mild acid elution, after which JY cells were pulsed with the peptide of interest in the presence of µ2M. Subsequently, expression levels of stable HLA-A^{*}0201 molecules were measured after designated incubation times.
Two of 13 high-affinity binding peptides showed a high off-rate from HLA-A*0201, because less than 10% of HLA-A^{*}0201-peptide complexes were detectable after 2 h incubation at 37°C. For all other peptides was the time required to decay for 50% (DT50) 2.5 h or longer (table 2). Interestingly, ALLERASATL (aa. 371-380) displayed high stability once complexed with HLA-A^{*}0201 (table B), rendering this peptide a potential CTL epitope with respect to binding characteristics despite the only intermediate binding capacity in the competition binding assay (table 1).

### In vitro proteasome mediated digestions of 27-mer polypeptides encompassing HLA-A^{*}0201 binding peptides

The two most important requirements for a peptide to be naturally presented on the cell surface are:
(i) excision from the protein by antigen processing and
(ii) sufficient binding affinity for HLA molecules. Therefore, we decided to perform, subsequent to characterization of HLA binding capacity, in vitro proteasome mediated digestions of 27-mer polypeptides encompassing aa.-sequences identified to bind with high and intermediate binding to HLA-A^{*}0201.
This analysis not only allowed assessment of precise C-terminal cleavage of putative epitopes but also evaluation of premature destruction of the epitope through a possible major proteolytic cleavage site within the epitope, as observed by us in a variant viral sequence. Digestion patterns of four 27-mer polypeptides are shown and discussed in detail (table C), because peptides for in vitro CTL inductions were chosen based on those results.
The 10-mer ALYVDSLFFL (aa. 300-309), which performed best in the binding assay, has its C-terminal leucine in common with the nonamer LYVDSLFFL (aa. 301-309). As the nonamer is described to be naturally presented in HLA-A24, it may be assumed that the C-terminal leucine is liberated during antigen processing. This is most likely accomplished by proteasome mediated cleavage, because C-terminal trimming as opposed to N-terminal trimming has never been observed. To confirm this, in vitro digestions of a 27-mer encompassing the decamer with its naturally flanking amino acids (PRAME 290-316) was performed by 20S proteasomes isolated from an human EBV transformed B cell line.
Moreover, this analysis aimed to asses whether the decamer was not destroyed by a major proteasomal cleavage site between its N-terminal alanine (aa. 300) and leucine (aa. 301), which would render the decamer not available for presentation by HLA-A*0201.
The 27-mer was incubated for 1 h, 4 h and 24 h at 37°C with the isolated proteasome and the mass spectrometry profile of the digestion product was analysed in detail (Table C1). As might be expected, a major cleavage site after leucine-309 was observed, because digestion fragments sharing this C-terminus were abundantly generated after 1 h incubation. Furthermore, the decamer (aa. 300-309) was not destroyed by a major cleavage site between residue 300 and 301. Instead, leucine-298 and glutamine-299 were generated as N-terminal residues after 1 h incubation. These results suggest that in vivo the 12-mer LQALYVDSLFFL (aa. 298-309) and the 11-mer QALYVDSLFFL (aa. 299-309) may be produced by the proteasome. The decamer (aa. 300-309) and the HLA-A24 presented nonamer (aa. 301-309) itself were found among the_digestion fragments, although only after 24 h incubation at low quantities. Finally, FLSLQCLQAL (aa. 292-301) was not C-terminally generated and_SLQCLQALYV (aa. 294-303) was not found as intact fragment, indicating that_these HLA-A^{*}0201 binding peptides are not likely to be naturally generated_in the processing pathway. The digestion pattern of 27-mer aa. 415-441 was determined because it_harbors peptides binding with high or intermediate affinity to HLA-A^{*}0201 (Table C2). A major cleavage site was observed between residues 433 and 434, because QSLLQHLIGL (aa. 424-433) and the complementary fragment aa. 434-441 were generated abundantly after 1 h digestion. The N-terminally elongated precursor (aa. 424-433) of high affinity binding nonamer aa. 425-433 was efficiently generated. The N-terminal counterpart aa. 415-423 was abundantly present as well, indicating a second major cleavage site after leucine-423. The other HLA-A^{*}0201 binding peptides (aa. 419-427, aa. 422-430 and aa. 422-431) were not or not efficiently liberated from the 27-mer polypeptide. Only after 4 h incubation fragment aa. 421-431 harboring PRAME 422-431 was found in low quantities. Table C3 shows the digestion pattern of PRAME 98-124 which harbors several peptides binding with moderate affinity in HLA^{*}A201. The 11-mer aa. 98-108 was the most abundantly generated fragment after 1 h, indicating that both VLDGLDVLL (aa. 1001-108) and AVLDGLDVLL (aa. 99-108) may be naturally_presented in vivo. Additionally, fragment aa. 98-109, harboring VLDGLDVLLA, was gerenated after 1 h incubation. Therefore, the latter peptide may be generated in vivo as well. To the contrary, fragments containing AVLDGLDVL (aa. 99-107) or GLDVLLAQEV (aa. 103-112) were not found. Finally, the 27-mer 359-385, containing HLA-A^{*}0201 binding peptides MLTDVSPEPL (aa. 360-369) and ALLERASATL (aa. 371-380), was in vitro cleaved by the proteasome. The results (Table C4) show that the first peptide is not generated, because fragments containing leucine position 369 as C-terminus were not found. However, fragments sharing leucine position 380 as C-terminus and the complementary fragment with position 381 as N-terminus were already found after 1 h, indicating a frequently cleaved site between residues 380 and 381. The exact sequence ALLERASATL was already generated after 1 h digestion and still present after 4 h and 24 h incubation, indicating this sequence as potential CTL epitope. A concise representation of digestion analysis of above discussed 27-mers and 27-mer polypeptides harboring the other high affinity binding peptides is shown in table D.
Summarizing, 5 HLA-A^{*}0201 binding peptides were not C-terminally excised by the proteasome. Furthermore, 5 peptides were C-terminally excised by a minor cleavage site, but these peptides (or their N-terminal elongated precursors) were either not generated (SLQCLQALYV and SISALQSLL) or generated in low quantities and/or after longer than 1 h incubation. Finally, only 5 peptides were C-terminally excised by a major cleavage site and found in correct length (variants), indicating possible CTL-epitope (precursor) peptides. Three of these peptides (SLYSFPEPEA, aa. 142-151; SLLQHLIGL, aa. 425-433 and VLDGLDVLL, aa. 100-108) and/or their N-terminal elongated precursors were already found after 1 h digestion in significant quantities.

### In vitro human CTL inductions against five putative HLA A*0201 restricted epitopes using PBMC of healthy donors

From the binding assays and the in vitro proteasome-mediated digestions, four peptides that were predicted to be naturally processed and presented were selected for in vitro CTL inductions. Separate CTL induction were performed against ALYVDSLFFL (aa. 300-309), SLLQHLIGL (aa. 425-433), VLDGLDVLL (aa. 100-108) and ALLERASATL (aa. 371-380) (in order of HLA-A^{*}0201 binding affinity). To optimally use all antigen presenting cells present in PBMC, we developed an in vitro human CTL induction protocol that used a mix of activated B cells39 and mature DC47-50 as APC in the primary induction. The peptide-loaded and irradiated APC-mix was cocultured with CD8+ autologous T cells from an HLA-A^{*}0201+ healthy donor as responder population in the presence of IL-7 and IL-12. At day 7 CTL cultures were restimulated with autologous activated B cells. Irradiated and peptide-pulsed restimulator cells were co-cultured with the responder population under addition of IL-7 and IL-12. Restimulations were repeated at day 14 and day 21 with the same restimulator cells in the presence of IL-2 and IL-12. At day 28 the bulk cultures were tested in a ⁵¹Cr release assay to asses peptide specificity. The CTL culture raised against SLLQHLIGL (aa. 425-433) showed a high specificity for targets loaded with this peptide, whereas the other three CTL cultures displayed only slightly increased lysis of targets loaded with the relevant peptide [data not shown].

### Peptide reactivities of CTL clones raised against PRAME 300-309, 425-433, 100-108 and 371-380

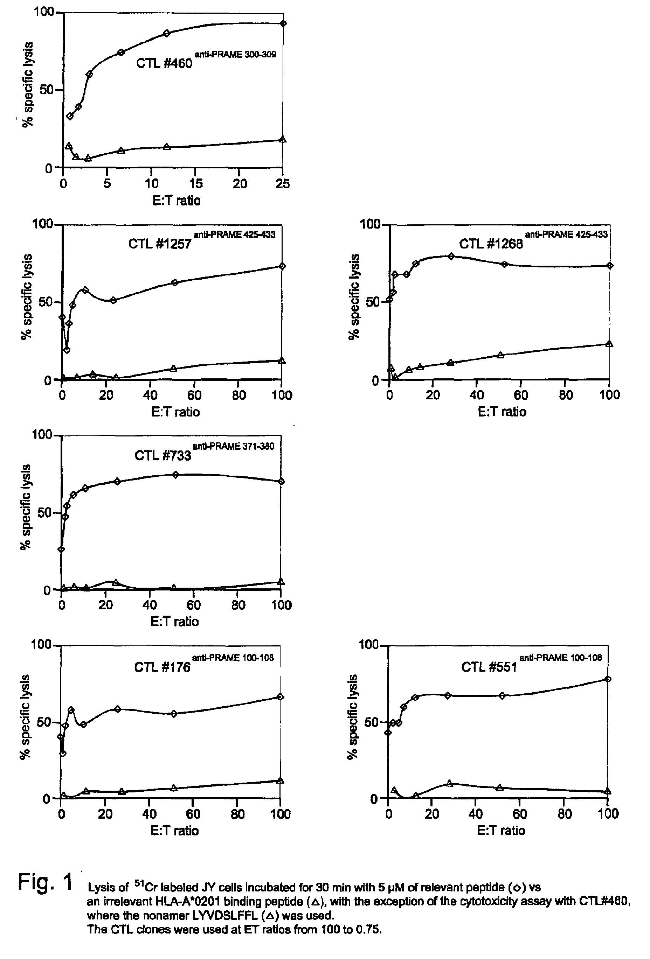

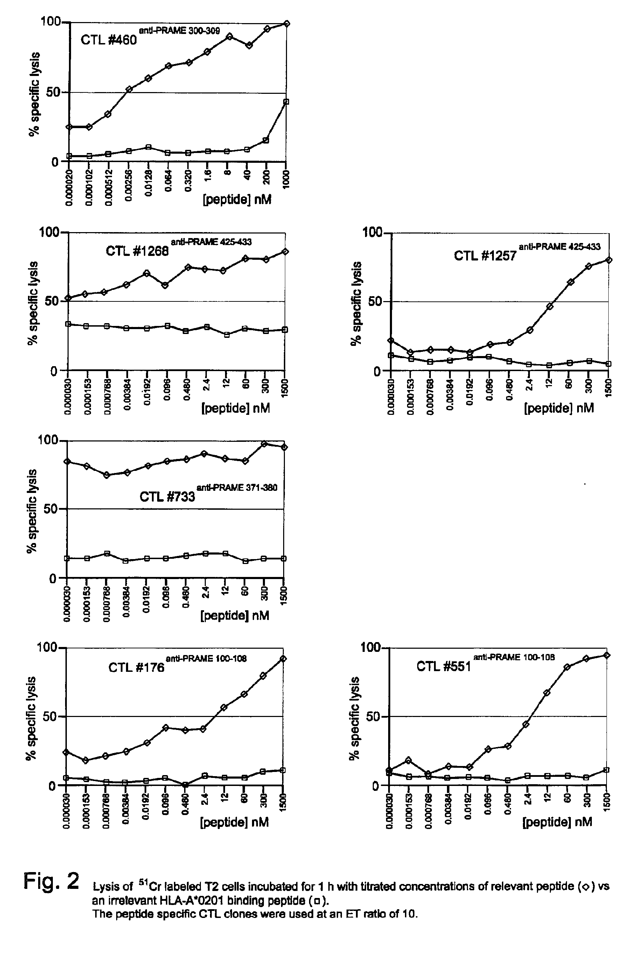

To isolate peptide specific CTL clones the four CTL cultures were cloned by limiting dilution at day 29. The peptide specificity of generated clones was assessed in a cytoxicity assay against target cells loaded with 5 µM of the relevant peptide versus an irrelevant HLA-A^{*}0201 binding peptide. Remarkably, despite low peptide-specificity of CTL bulk cultures against ALYVDSLFFL, VLDGLDVLL and ALLERASATL, CTL clones specific for all four peptides were found. This may be explained by the large amounts of CTL clones for each peptide specificity that were assayed (between 576 and 202), allowing isolation of peptide specific clonal CTL that constituted only a minor fraction of the bulk culture.
In summary, 9% of CTL clones raised against ALYVDSLFFL displayed peptide-specificity (19 of 202), 9% of clones induced against VLDGLDVLL showed specific lysis of peptide pulsed targets (51 of 576), 23 of 360 clones (6%) raised against ALLERASATL were peptide-specific, and finally, as may be expected a larger percentage namely 29% (97 of 336) of the clones grown from the bulk culture against SLLQHLIGL reacted peptide-specific.
Based on peptide specificity and affinity, growth characteristics and recognition of endogenous processed antigen, one or more CTL clones for each peptide specificity were chosen for a detailed functional characterization. At first, peptide specificity with targets pulsed at high peptide concentrations (5 (M) was examined. CTL clone #460, anti-ALYVDSLFFL, specifically lysed peptide pulsed targets at ET ratios as low as 0.5 and showed only very low crossreactivity with targets pulsed with the nonamer LYVDSLFFL (figure 1), which is naturally presented in HLA-A24 and displayed, despite not fullfilling the binding motif, intermediated binding affinity for HLA-A2^{*}0201 (data not shown). Likewise, anti-SLLQHLIGL CTL clones #1257 and #1268 showed specific lysis of T2 cells pulsed with the relevant peptide.
The CTL clones #176 and #551, which were raised against VLDGLDVLL, recognized their inducing peptide very efficiently. Finally, anti-ALLERASATL directed CTL clone #733 was able to lyse targets pulsed with the relevant peptide as well (figure 1).
Subsequently, peptide sensitivity of the CTL clones was determined in peptide titration experiments at a fixed ET ratio of 10 (fig. 2). CTL #460 was extremely sensitive in lysing T2 cells pulsed with the decamer ALYVDSLFFL: half maximal lysis was reached at about 3 pM peptide concentration. CTL clones #1257 and #1268 were able to half-maximally lyse targets loaded with SLLQHLIGL at 12 nM and less than 60 pM respectively. The synthetic peptide ALLERASATL was extremely well recognized by CTL #733, because at the lowest peptide concentration targets were still specifically lysed for 80%. Finally, CTL #176 and #551 displayed almost equal sensitivity for VLDGLDVLL: half-maximal lysis was reached at about 5 nM peptide concentration.
To analyse clonality of the CTL clones under investigation, we performed rtPCR analysis with a panel of 24 primers of junctional regions of TCRB transcripts from 22 well-established.TCRBV families to determine Vβ-usage of the T cell receptor. All CTL clones were shown to use a single Vβ, confirming clonality of the clones (data not shown).

### Peptide specific CTL clones specifically recognize an HLA-A^{*}0201+ renal cell carinoma cell line stably transfected with PRAME

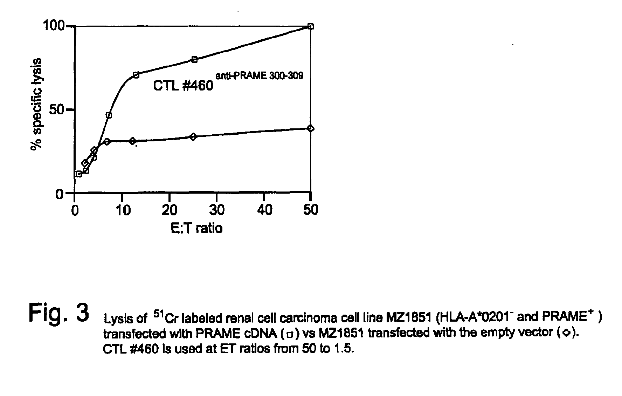

Endogenous presentation of ALYVDSLFFL in HLA-A^{*}0201 was explored by assessing the ability of CTL clone #460 to specifically recognize PRAME expresssing targets after transfection. For this purpose, the renal-cell carcinoma cell line MZ1851, which is HLA-^{*}0201+ but lacks PRAME expression, was stably transfected with fulllength PRAME cDNA (MZ1851-PRAME). As negative control MZ1851 was transfected with the empty vector (MZ1851-empty). PRAME expression was confirmed with rtPCR (data not shown). CTL #460, raised against the decamer PRAME 300-309 specifically lysed the PRAME expressing transfectant MZ1851-PRAME in 51Cr release assays (Fig. 3). This result indicates that PRAME 300-309 is endogenously processed and presented in HLA-A^{*}0201.

### CTL reactive with four PRAME peptides lyse a broad array of tumor cell lines expressing HLA-A^{*}0201 and PRAME

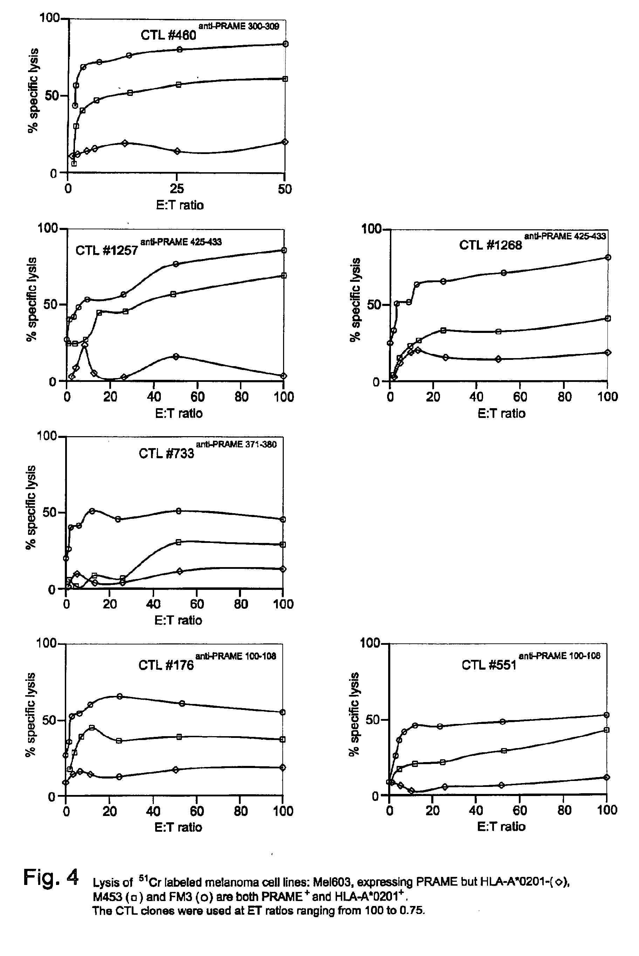

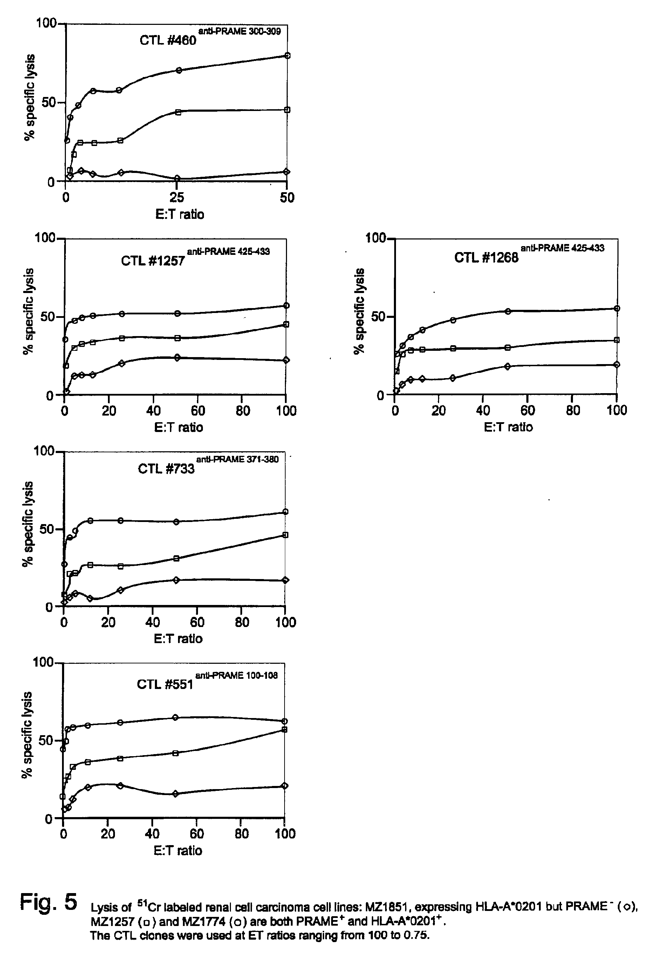

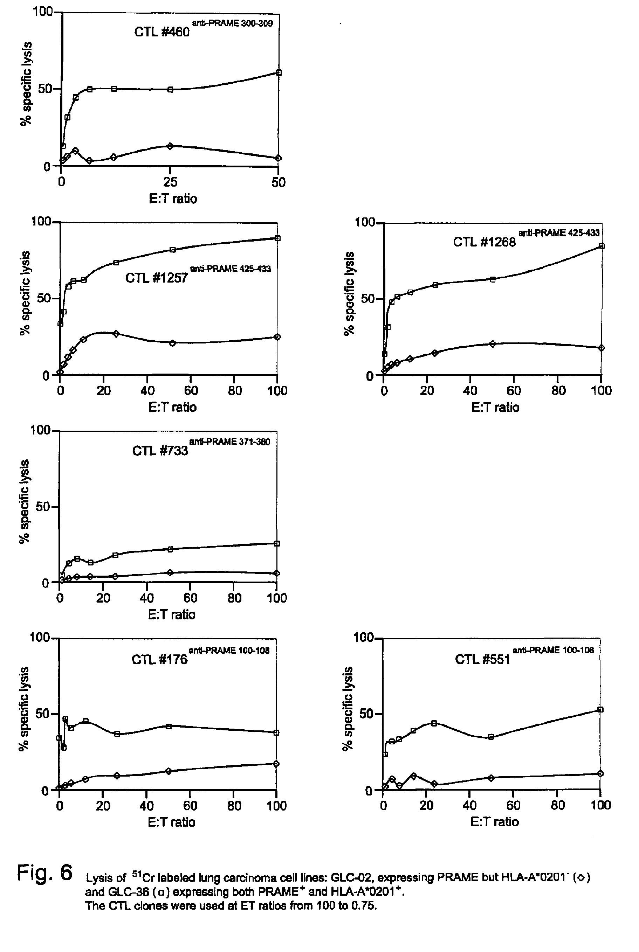

To investigate HLA-A^{*}0201 restricted presentation of PRAME 300-309, 424-450, 98-124 and 359-385 on tumor cells, we used panels of cell lines derived from various tumor types which have been reported to express PRAME (30;32). Lysis by the selected CTL clones of cell lines with or without HLA-A^{*}0201 expression and naturally expressing PRAME or lacking PRAME expression was compared. HLA-A^{*}0201 expression was confirmed by Facs analysis (data not shown) and PRAME expression by rtPCR or northern blotting (data not shown). The melanoma cell lines M453 and FM3, both expressing HLA-A^{*}0201 and PRAME+ were efficiently killed by CTL #460 (anti-ALYVDSLFFL), CTL #1257 and #1268 (anti-SLLQHLIGL), CTL #176 and #551 (anti-VLDGLDVLL) and CTL #733 (anti-ALLERASATL), whereas the melanoma cell line Me1603, which is PRAME+ but lacks HLA-A^{*}0201 expression, was not killed by these CTL clones (Fig. 4). These results confirm HLA-A^{*}0201 restriction of the CTL clones and indicate that the four peptides are naturally presented in HLA-A^{*}0201. Furthermore, lysis of the HLA-A^{*}0201+ renal-cell carcinoma (RCC) cell line MZ1851, which lacks PRAME expression, was compared with lysis of RCC cell lines MZ1257 and MZ1774, both expressing HLA-A^{*}0201 and PRAME. The selected CTL clones reactive against the four different PRAME peptides showed significant lysis of the two PRAME+ cell lines but not of MZ1851, confirming PRAME as source of target antigens (Fig. 5). Finally, lysis of lung carcinoma cell lines was HLA-A^{*}0201 restricted and PRAME specific as well, because only GLC-36 expressing HLA-A^{*}0201+ and PRAME+, and not GLC-02, which is PRAME+ but lacks HLA-A^{*}0201- expression was killed (Fig. 6).

Summarizing, we observed a consistent lysis by the CTL clones under investigation of tumor celllines when both the restricting MHC molecule and the tumor Ag were expressed. These results indicate that PRAME 300-309, 425-433, 100-108 and 371-380 are presented in the context of HLA-A^{*}0201 on a broad array of tumor cell lines encoded by human gene MAGE-3 and presented by HLA-A2 induces cytolytic T lymphocytes that recognize tumor cells expressing MAGE-3.

### REFERENCES

Coux, O., K. Tanaka, and A.L. Goldberg. 1996 Structure and functions of the 20S and 26S proteasomes. Annu. Rev. Biochem. 65: 801.
Rock, K.L., and A.L. Goldberg. 1999. Degradation of cell proteins and the generation of MHC class I-presented peptides. Annu. Rev. Immunol. 17: 739.
Brown, M.G., Driscoll, J., and Monaco, J.J. 1991. Structural and serological similarity of MHC-linked LMP and proteasome (multicatalytic proteinase) complexes. Nature 353: 355.
Ortiz-Navarrette, V., Seelig, A., Gernold, M., Frentzel, S., Kloetzel, P.M., and HÑmmerling, G.J. 1991 Subunit of the 20S proteasome (multicatalytic proteinase) encoded by the major histocompatibility complex. Nature 353: 662. Rivett, A.J. 1993. Proteasomes: multicatalytic proteinase complexes. Biochem. J. 291: 1.
Lîwe, J., Stock, D., Jap, B., Zwickl, P., Baumeister, W., and Huber, R. 1995. Crystal structure of the 20S proteasome from the archaon T. acidophilum at 3.4 è resolution. Science 268: 533.
Hershko, A., and Ciechanover, A. 1992. The ubiquitin system for protein degradation. Annu. Rev. Biochem. 61: 761.
Rechsteiner, M., Hoffman, L., and Dubiel, W. 1993. The multicatalytic and 26S proteases. J. Biol. Chem. 268: 6065.
Murakami, Y., Matsufuji, S., Kameji, T., Hayashi, S.-I., Igarashi, K., Tamura, T., Tanaka, K., and Ichihara, A. 1992. Ornithine decarboxylase is degraded by the 26S proteasome without ubiquitin. Nature 360: 597.
Gerards, W.L.H., de Jong, W.W., Boelens, W., and Bloemendal, H. 1998. Structure and assembly of the 20S proteasome. Cellular and Molecular Life Sciences 54: 253.
Leibovitz, D., Koch, Y., Fridkin, M., Pitzer, F., Zwickl, P., Dantes, A., Baumeister, W., and Amsterdam, A. 1995. Archarbacterial and eukaryotic proteasomes prefer different sites in cleaving gonadotropin-releasing hormone. J. Biol. Chem. 270: 11029.
Cardozo, C., Chen, W.-E., and Wilk, S. 1996. Cleavage of Pro-X and Glu-X bonds catalyzed by the branched chain amino acid preferring activity of the bovine pituitary multicatalytic proteinase complex (20S proteasome). Arch. Biochem. Biophysics. 334: 113.
Gaczynska, M., K.L. Rock, and A.L. Goldberg. 1993. Gamma-interferon and expression of MHC genes regulate peptide hydrolysis by proteasomes. Nature 365: 264.
Boes, B., Hengel, H., Ruppert, Th., Multhaup, G., Koszinowski, U.H., and Kloetzel, P.-M. (1994) Interferon stimulation modulates the proteolytic activity and cleavage site preference of 20S mouse proteasome. J. Exp. Med. 179, 901-909.
Griffin, T.A., D. Nandi, M. Cruz, H.J. Fehling, L.V. Kaer, J.J. Monaco, and R.A. Colbert. 1998. Immunoproteasome assembly: cooperative incorporation of interferon (-inducible subunits. J. Exp. Med. 187:97.
Gausepohl, H., Kraft, M., Boulin, C., and Frank, R.W. (1990). Automated multiple peptide synthesis with BOP activation. In Proceedings of the eleventh American peptide Symposium, J.E. Rivier and G.R. Marshall, eds. (ESCOM, Leiden), p 1003.
Groettrup, M., Ruppert, T., Kuehn, L., Seeger, M., Standera, S., Koszinowski, U., Kloetzel, P.-M. (1995) The interferon- inducible 11S regulator (PA28) and the LMP2/LMP7 subunits govern the peptide production by the 20S proteasome in vitro. J. Biol. Chem. 270, 23808-23815.
Eggers, M., Boes-Fabian, B., Ruppert, T., Kloetzel, P.-M., and Koszinowski, U.H. (1995) The cleavage preference of the proteasome governs the yield of antigenic peptides. J. Exp. Med. 182, 1865-1870.
Traversari, C., van der Bruggen, P., van den Eynde, B., Hainaut, P., Lemoine, C., Ohta, N., Old, L., and Boon, T. (1992) Transfection and expression of a gene coding for a human melanoma antigen recognized by autologous CTL. Immunogenetics. 35, 145-152.
Ossendorp, F., M. Eggers, A. Neisig, T. Ruppert, M. Groettrup, A. Sijts, E. MengedÇ, P-M. Kloetzel, J.J. Neefjes, U. Koszinowski, and C. Melief. 1996. Asingle residue exchange within a viral CTL epitope alters proteasome-mediated degradation resulting in lack of antigen presentation. Immunity, 5:115.
Neisig, A., J. Roelse, A.J.A.M. Sijts, F. Ossendorp, M.C.W. Feltkamp, W.M. Kast, C.J.M. Melief, and J.J. Neefjes. 1995. Major differences in transporter associated with antigen presentation (TAP) -dependent translocation of MHC class I-presentable peptides. J. Immunol. 154:1273.
Sijts, A.J.A.M., M.L. de Bruijn, M.E. Ressing, J.D. Nieland, E.A.M. MengedÇ, C.J. Boog, F. Ossendorp, W.M. Kast, and C.J.M. Melief. 1994. Identification of an H-2Kb-presented Moloney murine leukemia virus cytotoxic T lymphocyte epitope that displays enhanced recognition in H-2 Db mutant bml3 mice. J. Virol. 68:6038.
Kast, W.M., L. Roux, J. Curren, H.J.J. Blom, A.C. Voordouw, R.H. Meloen, D. Kolakofski, and C.J.M. Melief. 1991. Protection against lethal Sendai virus infection by in vivo priming of virus-specific cytotoxic T lymphocytes with an unbound peptide. Proc. Natl. Acad. Sci. USA. 88:2283.
Theobald, M., T. Ruppert, U. Kuckelkorn, J. Hernandez, A. HNussler, E.A. Ferreira, U. Liewer, J. Biggs, A.J. Levine, C. Huber, U.H. Koszinowski, P.M. Kloetzel, and L.A. Sherman. 1998. The sequence alteration associated with a mutational hotspot in p53 protects cells from lysis by cytotoxic T lymphocytes specific for a flanking peptide epitope. J. Exp. Med. 188:1017-1028.

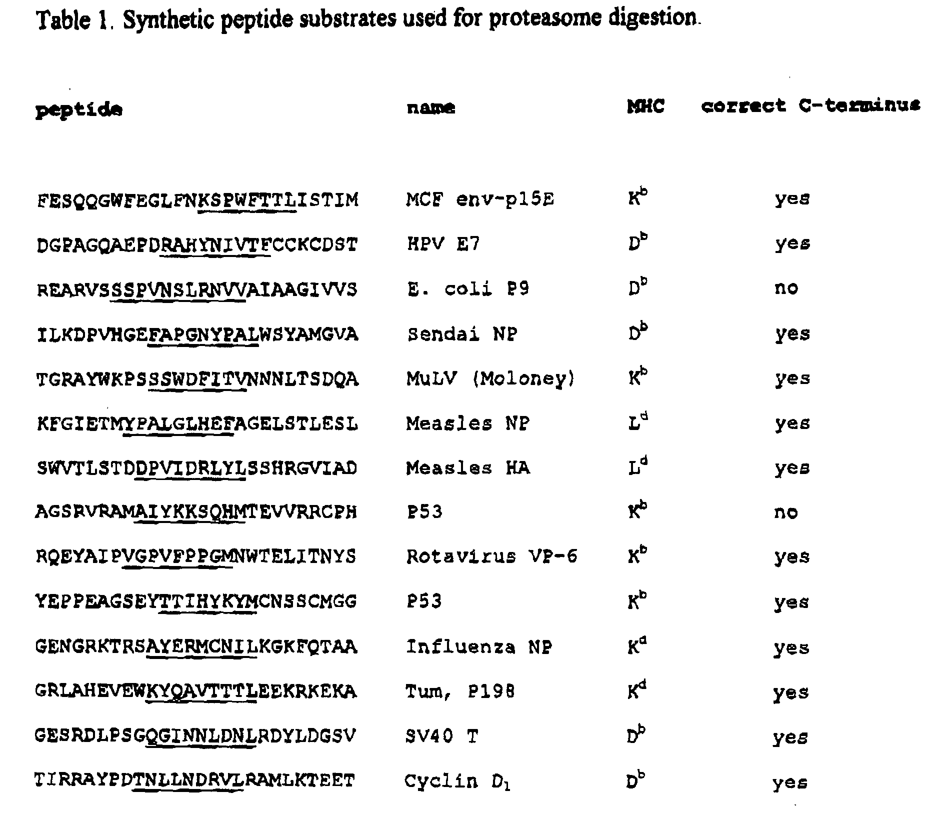

### CTL-epitopes are underlined with the C-terminus at the right end site of the underlined epitope.

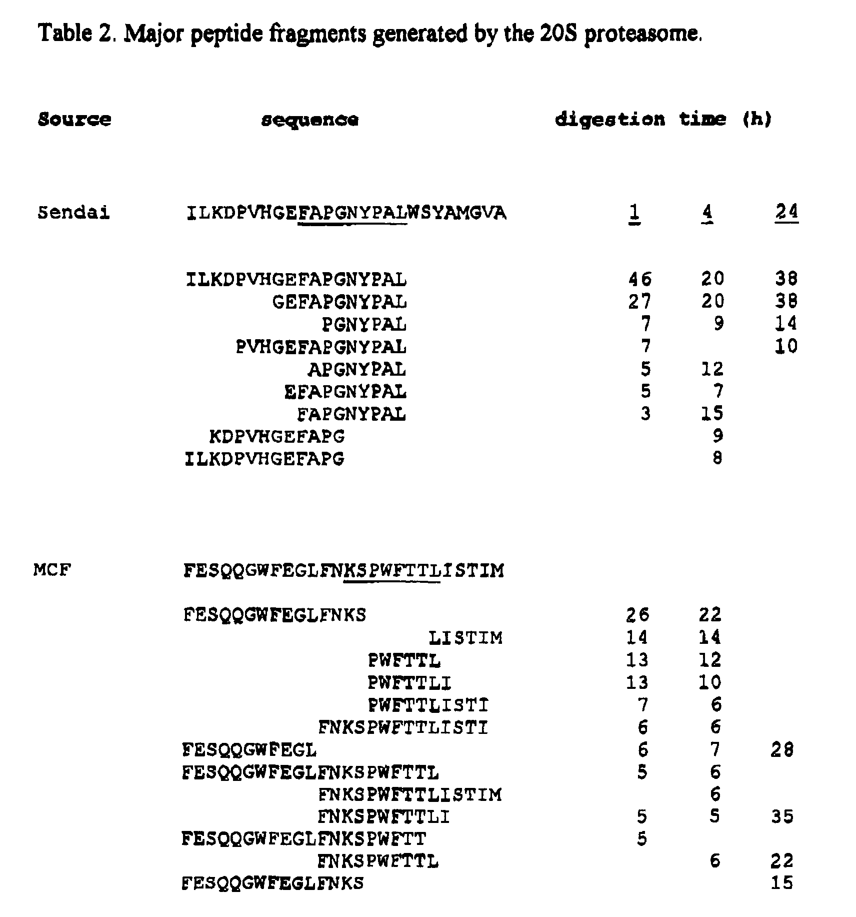

**The amount of peptide fragment is given as a relative percentage of the total intensity of the peptide fragments in the spectrum. The CTL-epitope is underlined.**

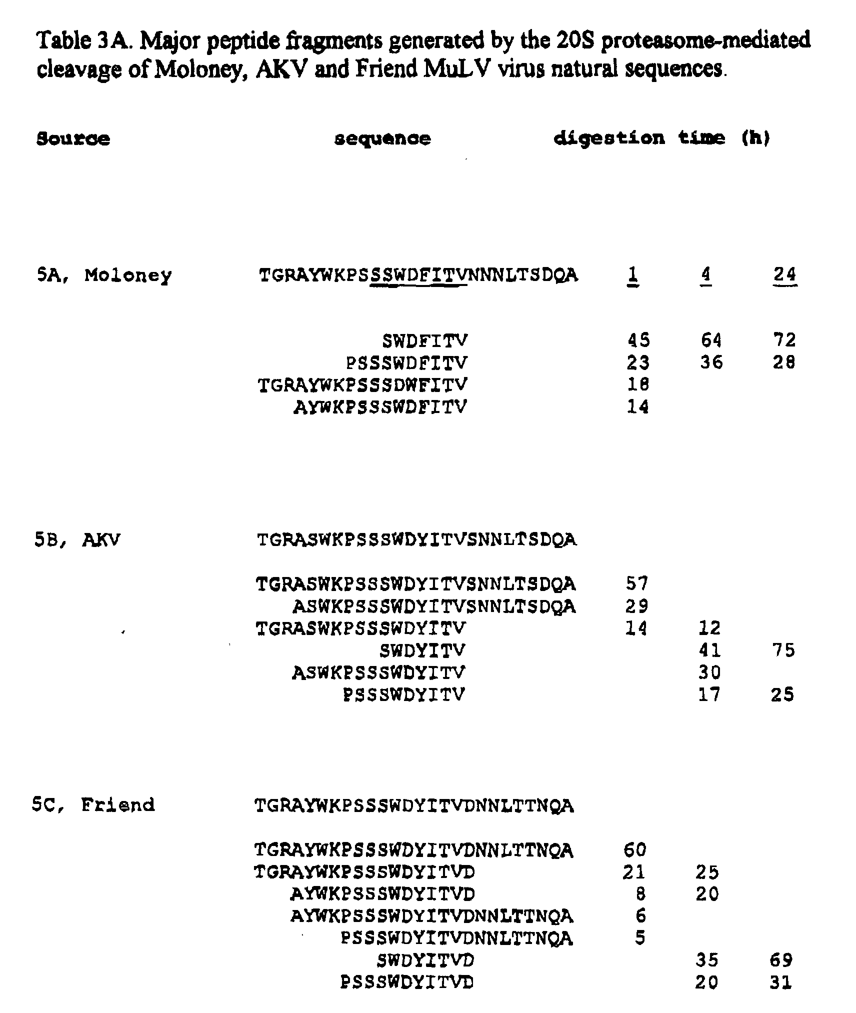

Intensity is given as a percentage of the total amount of intensity of the peak fragments depicted below.
Fragments with intensities less than three times the noise peak in a spectrum were ignored in the determination of sequences of peptides generated after digestion by 20S proteasomes.

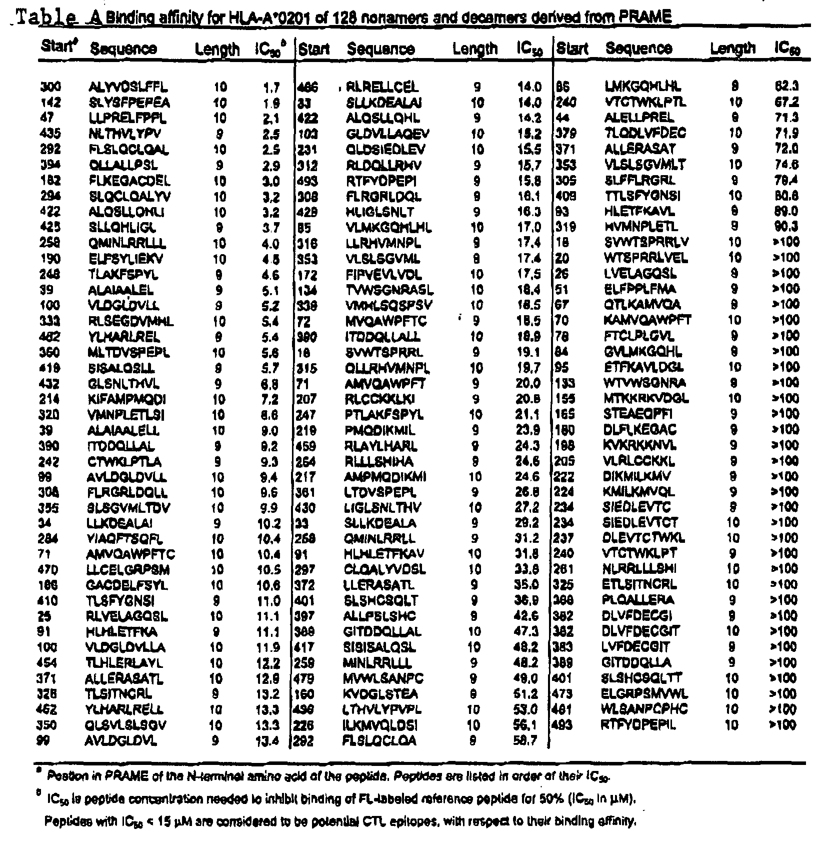

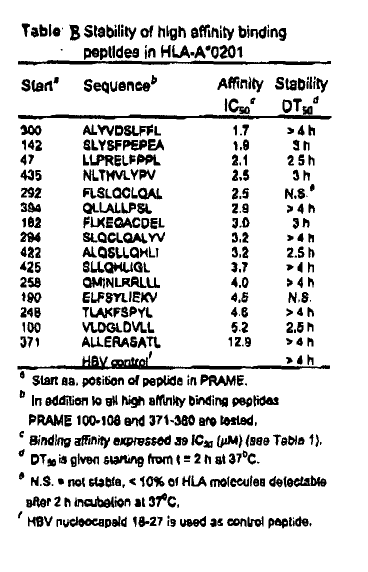

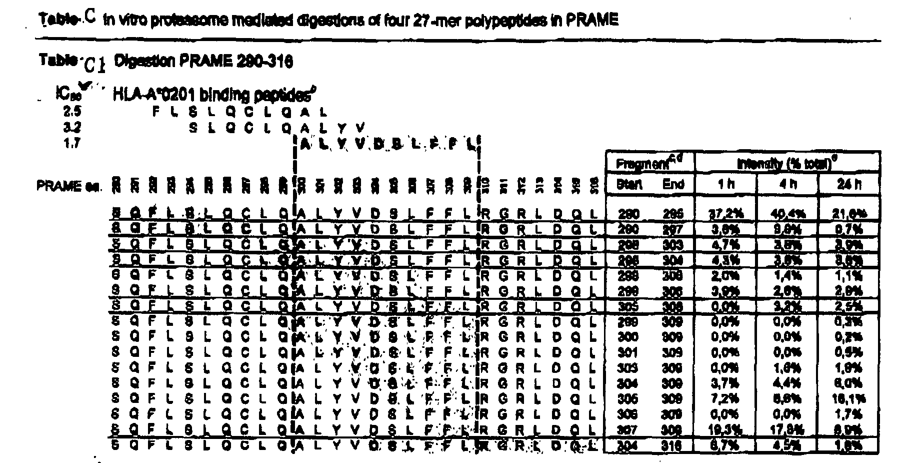

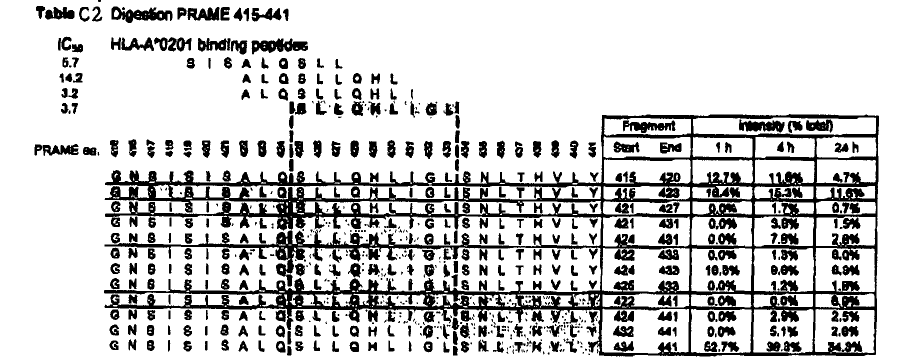

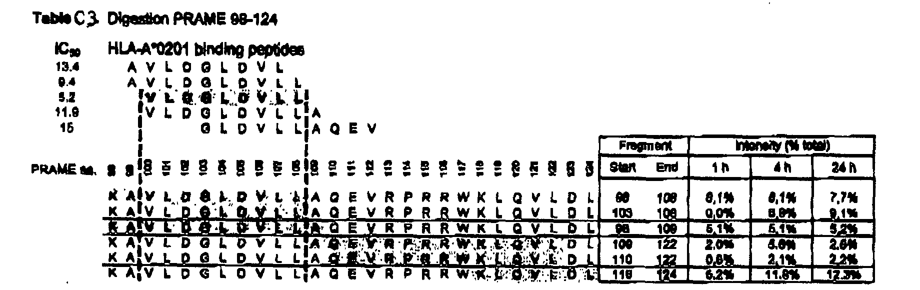

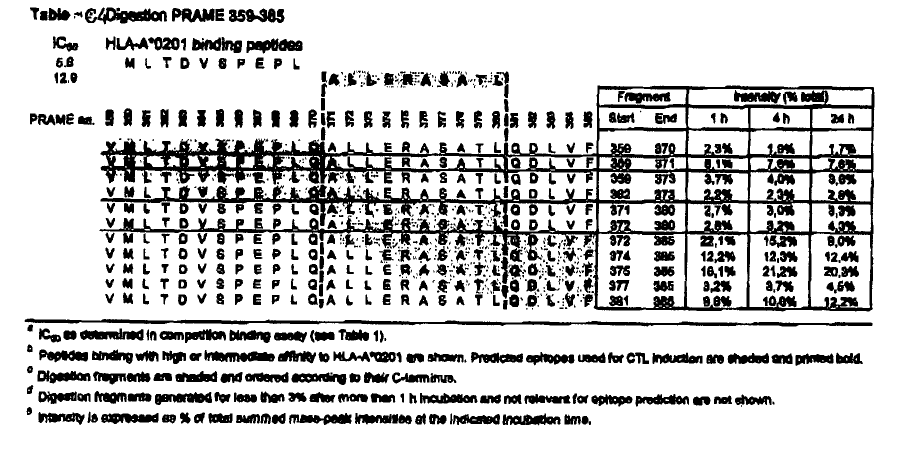

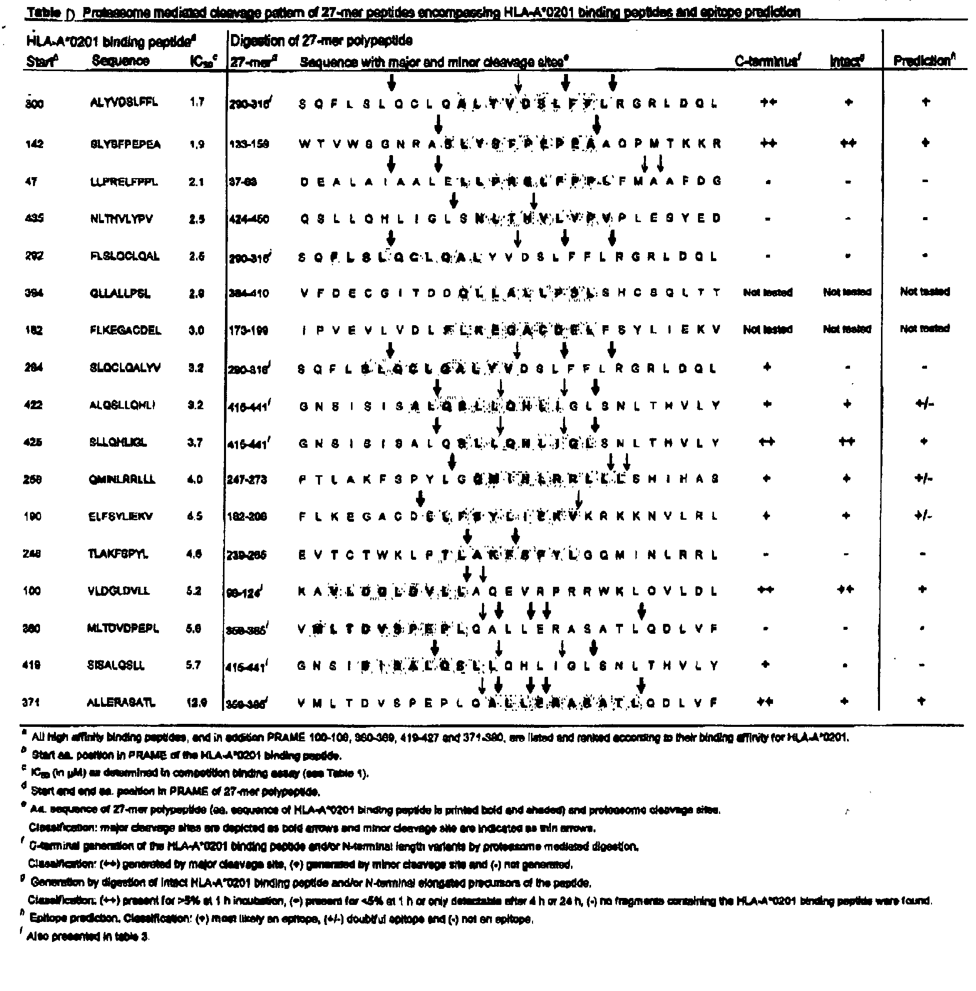

## Claims

1. A method for selecting and/or producing a T cell epitope, comprising subjecting a precursor peptide or polypeptide to the action of a 20S proteasome, in particular an IFN-γ or other immune-related proteasome or a functional equivalent thereof to determine the location of the c-terminus of said T cell epitope.

2. A method for selecting and/or producing according to claim 1 wherein said precursor peptide or polypeptide is tested for other T cell epitope characteristics.

3. A method according to claim 2, wherein said further testing comprises testing for the right anchor residues, the proper cleavage sites, the proper pathway signals, and/or the stability of the MHC-T cell epitope complex.

4. A T cell epitope obtainable by a method according to any one of claims 1-3.

5. A nucleic acid encoding at least one T cell epitope according to claim 4.

6. A nucleic acid according to claim 5 comprising at least two sequences encoding a T cell epitope, separated by at least one protelytic cleavage site.

7. A gene delivery vehicle comprising a nucleic acid according to claim 4 or 5.

8. A pharmaceutical composition comprising a gene delivery vehicle according to claim 7.

9. A pharmaceutical composition comprising at least one epitope according to claim 4.

10. A proteinaceous molecule obtainable by expression of a nucleic acid according to claim 5 or 6.

11. A pharmaceutical composition comprising a proteinaceous molecule according to claim 10.

12. A pharmaceutical composition according to claim 8, 9 or 11 further comprising an adjuvans.

13. A pharmaceutical composition according to claim 8, 9, 11 or 12 comprising an antigen presenting moiety.

14. A pharmaceutical composition according to claim 13 wherein said moiety comprsises a major histocompatibility molecule.

15. A pharmaceutical composition according to claim 14 wherein said major histocompatibility molecule is present on an antigen presenting cell, such as a dendritic cell.
